(19) Europäisches Patentamt
European Patent Office
Office européen des brevets

(11) **EP 2 279 264 B1**

(12) **EUROPÄISCHE PATENTSCHRIFT**

(45) Veröffentlichungstag und Bekanntmachung des Hinweises auf die Patenterteilung:
**19.08.2015 Patentblatt 2015/34**

(21) Anmeldenummer: **09769081.2**

(22) Anmeldetag: **20.05.2009**

(51) Int Cl.:
*C12Q 1/68* (2006.01)  *A01K 67/033* (2006.01)
*C12Q 1/66* (2006.01)

(86) Internationale Anmeldenummer:
**PCT/EP2009/056106**

(87) Internationale Veröffentlichungsnummer:
**WO 2009/156232 (30.12.2009 Gazette 2009/53)**

(54) **VERFAHREN ZUR CHARAKTERISIERUNG DER BIOLOGISCHEN AKTIVITÄT VON TRICHURIS-EIERN**

METHOD FOR CHARACTERISING THE BIOLOGICAL ACTIVITY OF TRICHURIS EGGS

PROCÉDÉ DE CARACTÉRISATION DE L'ACTIVITÉ BIOLOGIQUE D'OEUFS DE TRICHURIS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **21.05.2008 EP 08009344**

(43) Veröffentlichungstag der Anmeldung:
**02.02.2011 Patentblatt 2011/05**

(73) Patentinhaber: **Dr. Falk Pharma GmbH**
**79108 Freiburg (DE)**

(72) Erfinder:
- **TEWES, Bernhard**
**79108 Freiburg (DE)**
- **WILHELM, Rudolf**
**76476 Bischweier (DE)**

(74) Vertreter: **Keller, Günter et al**
**Lederer & Keller**
**Patentanwälte Partnerschaft mbB**
**Unsöldstrasse 2**
**80538 München (DE)**

(56) Entgegenhaltungen:
WO-A-02/066671    WO-A-2007/076868
WO-A-2007/134761

- **PECSON BRIAN M ET AL: "A real-time PCR method for quantifying viable ascaris eggs using the first internally transcribed spacer region of ribosomal DNA." APPLIED AND ENVIRONMENTAL MICROBIOLOGY DEC 2006, Bd. 72, Nr. 12, Dezember 2006 (2006-12), Seiten 7864-7872, XP002501996 ISSN: 0099-2240**
- **KRINGEL ET AL: "Trichuris suis population dynamics following a primary experimental infection" VETERINARY PARASITOLOGY, ELSEVIER SCIENCE, AMSTERDAM, NL, Bd. 139, Nr. 1-3, 30. Juni 2006 (2006-06-30), Seiten 132-139, XP005460788 ISSN: 0304-4017 in der Anmeldung erwähnt**
- **STINEAR T ET AL: "DETECTION OF A SINGLE VIABLE CRYPTOSPORIDIUM PARVUM OOCYST IN ENVIRONMENTAL WATER CONCENTRATES BY REVERSE TRANSCRIPTION-PCR" APPLIED AND ENVIRONMENTAL MICROBIOLOGY, AMERICAN SOCIETY FOR MICROBIOLOGY, US, Bd. 62, Nr. 9, 1. September 1996 (1996-09-01), Seiten 3385-3390, XP002923031 ISSN: 0099-2240**
- **BEER R J S: "Studies on the biology of the life-cycle of Trichuris suis Schrank, 1788" PARASITOLOGY, CAMBRIDGE UNIVERSITY PRESS, LONDON, GB, Bd. 67, Nr. 3, 1. Dezember 1973 (1973-12-01), Seiten 253-262, XP008113104 ISSN: 0031-1820**
- **SUMMERS ET AL: "Trichuris suis therapy for active ulcerative colitis: A randomized controlled trial" GASTROENTEROLOGY, ELSEVIER, PHILADELPHIA, PA, Bd. 128, Nr. 4, 1. April 2005 (2005-04-01), Seiten 825-832, XP005110857 ISSN: 0016-5085**

Printed by Jouve, 75001 PARIS (FR)

(Forts. nächste Seite)

- JOHNSON P W ET AL: "An in-vitro test for assessing the viability of Ascaris suum eggs exposed to various sewage treatment processes." INTERNATIONAL JOURNAL FOR PARASITOLOGY APR 1998, Bd. 28, Nr. 4, April 1998 (1998-04), Seiten 627-633, XP008097926 ISSN: 0020-7519
- BOES J ET AL: "Embryonation and infectivity of Ascaris suum eggs isolated from worms expelled by pigs treated with albendazole, pyrantel pamoate, ivermectin or piperazine dihydrochloride" VETERINARY PARASITOLOGY, ELSEVIER SCIENCE, AMSTERDAM, NL, Bd. 75, Nr. 2-3, 28. Februar 1998 (1998-02-28), Seiten 181-190, XP002449735 ISSN: 0304-4017

**Beschreibung**

Die vorliegende Erfindung betrifft ein Verfahren zur Bestimmung der biologischen Aktivität von Helminth-Eiern, nämlich von *Trichuris* Eiern, bevorzugt *Trichuris suis* Eiern, in verschiedenen Stadien ihres Lebenszyklus. Das Verfahren ermöglicht, Präparationen von Helminth-Eiern, als wirksame Bestandteile therapeutischer Arzneimittel, kontrolliert herzustellen und eine sichere und therapeutisch effiziente Anwendung am Menschen zu gewährleisten.

Der Einfluss von Parasiteninfektionen auf die Aktivierung des Immunsystems ihrer tierischen Wirte ist bekannt (Review, D M McKay, Parasitology 2006, 132: 1-12). Eine solche Aktivierung beeinflusst auch das Auftreten und den Verlauf von Autoimmunkrankheiten. Epidemiologische Studien zeigen, dass in Regionen mit hohen Raten von Wurminfektionen Autoimmunerkrankungen seltener sind als in Regionen, in denen auf Grund besserer hygienischer Verhältnisse diese Infektionsraten niedriger sind. Cytokinprofile von Patienten mit Morbus Crohn, einer chronisch entzündlichen Darmerkrankung, haben gezeigt, dass Th2-Immunzellen durch Helminthen Infektionen stimuliert werden können. Morbus Crohn, eine Th1-dominierte Autoimmunerkrankung kann durch eine Helminthen Infektion verhindert bzw. beeinflusst werden (Summers et al., Am J Gastroenterol 2003, 98: 2034-2041).

Schon 1971 berichtete Beer (Br. Med. J., 1971, 3: 41), dass *Trichuris suis* ein geeigneter Nematode sein könnte, um eine gezielte, moderate Infektion des Menschen zu erreichen, ohne die pathogenen Effekte hervorzurufen, die beispielsweise mit einer Infektion durch den humanpathogenen Erreger *Trichuris trichiura* einhergehen. Nach neueren Studien scheint die Infektion des Menschen mit *Trichuris suis* nur transient zu sein und die Würmer werden offensichtlich abgestoßen, bevor sie sich vermehren können. Gleichwohl deuten die positiven klinischen Studien zum Einsatz von *Trichuris suis* bei chronisch entzündlichen Darmerkrankungen darauf hin, dass diese transiente Infektion des Menschen eine therapeutisch wirksame Modulation des Immunsystems auslösen kann (Summers et al., Am. J. Gastroenterol. 2003, 98: 2034-2041).

Der Lebenszyklus von *Trichuris suis* beginnt mit dem Absetzen der nicht-embryonierten Eier (LO-Stadium), die vom infizierten Tier mit den Faeces ausgeschieden werden. Im Boden erfolgt über einen Zeitraum von 3-6 Monaten die Embryonierung zum ersten Larven-Stadium L1. In den ersten Tagen nach Erreichen des L1-Stadium lassen sich in den Eiern Larven-Bewegungen verfolgen, danach verfallen die Eier in einen Ruhezustand, in dem sie über Jahre verharren können, ohne jedoch ihre Infektivität zu verlieren. Nach oraler Aufnahme durch einen passenden Wirt schlüpfen die L1-Larven im intestinalen Lumen aus dem Ei und dringen binnen weniger Stunden in die Mucosa des Caecums und Colons ein. In der intestinalen Mucosa durchlaufen die Larven über die nächsten Wochen weitere Stadien der Larval-Entwicklung (L2-L4), bis sie schließlich als adulte L5-Larven wieder in das intestinale Lumen austreten (Beer, Parasitol. 1973, 67: 253-262).

Für die pharmazeutische Herstellung von *Trichuris suis* Eiern (*Trichuris suis ova* = TSO), werden nicht-embryonierte TSO (L0), die entweder *in vitro* oder *in situ* abgelegt wurden, isoliert und mit geeigneten Methoden aufgereinigt (WO 1999 33479, WO 2007 076868, WO 2007 134761). Danach erfolgt die Embryonierung unter kontrollierten Laborbedingungen zu biologisch aktiven (L1) TSO, die den pharmazeutisch aktiven Bestandteil des Arzneimittels darstellen.

Kringel et al. (Veterinary Parasitology (2006), Seiten 132-139)) beschreiben die Dynamik einer *Trichuris suis*-Population nach einer ersten experimentellen Infektion von Schweinen, aber keine Verfahren zur Bestimmung der Lebensfähigkeit von embryonisierten *Trichuris suis-Eiern.*

Johnson et al. (Int. J. for Parasitology (1998), Seiten 627-633)) beschreiben ein *in vitro*-Testverfahren zur Bestimmung der Lebensfähigkeit von *Ascaris suum*-Eiern (Schweine-Spulwurm), nicht aber von *Trichuris suis*-Eiern. Beschrieben wird, wie widerstandsfähig *Ascaris suum-Eier* bei der Behandlung von Abwasser sind.

Pecson et al. (Applied and Environmental Microbiology (2006), Seiten 7864-7872)) beschreiben eine Real Time PCR zur Bestimmung von lebensfähigen *Ascaris*-Eiern. Das Prinzip dieser Methode ist die Quantifizierung der Kopiezahl der Gene der ribosomalen DNA. Die Anzahl der Genkopien ist proportional zu der Anzahl der *Ascaris*-Zellen in dem Wurmei. Wenn sich die Eier aus einer einzelnen Zelle zu einer reifen Larve entwickeln, steigt die Anzahl der Genkopien mit jeder Zellteilung an, bis ein konstantes Niveau pro Ei erzielt ist. Die Methode beruht also auf der Annahme, dass sich nur lebensfähige *Ascaris*-Eier aus dem Einzelzellstadium zu Larven entwickeln. Eine Unterscheidung zwischen lebensfähigen und bereits entwickelten, aber abgestorbenen Eiern ist nicht möglich.

Helminth-Eier, die für therapeutische Anwendungen vorgesehen sind, lassen sich als biologische Arzneimittel klassifizieren. Ein zentraler Parameter, der bei Herstellung und Anwendung biologischer Arzneimittel getestet werden muss, ist die biologische Aktivität, welche letztendlich ein Maß für die therapeutische Wirksamkeit des Arzneimittels ist. Ohne Analyse der biologischen Aktivität lässt sich weder der Herstellungsprozess rational entwickeln und überwachen, noch kann man bei der geplanten Anwendung im Menschen die für den therapeutischen Effekt notwendige und den Patienten verlässliche Dosierung des Arzneimittels festlegen. Aus diesem Grunde lassen sich nur solche Helminth-Ei-Präparationen sicher und effektiv als Arzneimittel einsetzen, deren biologische Aktivität hinreichend qualifiziert ist.

Bisher stehen für *Trichuris suis* drei analytische Verfahren zur Verfügung, die die biologische Aktivität jedoch nur unzureichend charakterisieren:

1. Die Bestimmung der Embryonierungsrate (Kringel et al., Vet. Parasitol. 2006, 139: 132-139, Absatz 2.2 *(Trichuris suis)*; auch Johnson et al. Intl. J. Parasitol. 1998, 28, 627-633 (Ascaris suum)): Die Untersuchung erfolgt mikroskopisch. Anhand morphologischer Kriterien wird beurteilt, ob die Eier eine intakte, vollständig ausgebildete L1-Larve enthalten. Aus der morphologischen Beurteilung allein lässt sich jedoch im Gegensatz zu der in dieser Anmeldung dargestellten molekularbiologischen Methode nicht zweifelsfrei ableiten, ob die Larven tatsächlich vollständig embryoniert sind. Eine weitere Limitierung liegt darin, dass die Embryonierungsrate keine Aussage über die Viabilität und biologische Aktivität der embryonierten L1-TSO bietet. Zudem bedarf die morphologische Beurteilung der Erfahrung des Beobachters und die subjektive Einordnung von morphologischen Grenzfällen limitiert die Richtigkeit und Präzision dieser Methode.

2. Die Bestimmung der Infektionsrate: Für *Trichuris suis* erfogt die Untersuchung im Schwein. Zu einem bestimmten Zeitpunkt nach Infektion wird die Anzahl der in der Darmschleimhaut etablierten Larven bestimmt und in Relation zur applizierten, Dosis an TSO gesetzt (Kringel et al., Vet. Parasitol., 2006, 139: 1,32-139, auch Summers et al., 2005, Gastroenterology, 128: 825-832 oder Johnson et al. Intl. J. Parasitol. 1998, 28, 627-633 (Ascaris suum)). Problematisch an der Bestimmung der Infektionsrate ist, dass sie nicht allein von der Funktionalität der Helminth-Eier, sondern gleichermaßen auch von individuellen Wirtsfaktoren, wie beispielsweise dem Immunsystem, der Darmflora und der Darmfunktion der Versuchstiere abhängt. Die Bestimmung der Infektionsrate im Versuchstier ist also intrinsisch mit einer hohen Variabilität verbunden und lässt sich aufgrund des natürlichen Testsystems nicht standardisieren, welches die Eignung als biologischer Aktivitätstest stark einschränkt. Für *Trichuris suis* ist der Test der Infektivität im Schwein mit einer langen Inkubationszeit von mindestens drei Wochen und aufgrund der hohen Variabilität mit einer hohen Tierzahl verbunden, was einen routinemäßigen Einsatz dieses Tests sowohl ethisch wie auch wirtschaftlich fragwürdig erscheinen lässt.

Weiterhin beschreibt Kringel et al. die Rückgewinnung von Eiern aus dem Kot der Tiere als Nachweis für die biologische Aktivität der Helminth-Eier, mit denen die Tiere infiziert wurden. Dazu wartet man 7-8 Wochen nach Infektion der Tiere, bis die geschlüpften Larven zum L5-Stadium heranreifen, sich paaren und ihrerseits Eier absetzen. Es gibt keinen direkten quantitativen Zusammenhang, aus dem sich von der Anzahl der Eier im Kot auf die Infektionsrate oder die biologische Aktivität der adulten Larven schliessen ließe. Die Methode von Kringel et al. ist deutlich von der in dieser Anmeldung beschriebenen Bestimmung der Schlüpfrate in vivo zu unterscheiden, da bei Kringel et al.. die Eier der nächsten Generation untersucht werden.

3. Die WO 2007/134761 beschreibt ein Verfahren zum Nachweis der Viabilität von *Trichuris suis-Eiern,* bei dem der Durchgang der Eier durch die Magen-Darm-Passage eines Schweines in vitro simuliert wird und man dadurch die Larven zum Schlüpfen bringt.

Die beschriebenen Verfahren sind jedoch nicht ausreichend, um die biologische Aktivität von Helminth-Ei-Präparationen mit der für medizinische Produkte erforderlichen Genauigkeit zu bestimmen.

Eines der Hauptprobleme eines biologischen Arzneimittels, dessen biologische Aktivität im Tier getestet wird, ist also die Standardisierung des Präparats. Der Einsatz von Tiermodellen, wie die oben erwähnte Infektion von Schweinen, ist äußerst aufwändig und nimmt eine erhebliche Zeit in Anspruch. Im Rahmen der vorliegenden Erfindung wird daher ein Verfahren beschrieben, bei dem verschiedene Arten von Tests durchgeführt werden, die unterschiedliche Aspekte der Entwicklung der Helminth-Eier und ihre biologische Aktivität betreffen. Um zu einer verlässlichen Aussage zu gelangen, muß bei der zu untersuchenden Charge wenigstens einer der erfindungsgemäßen Tests durchgeführt werden, bevorzugt werden jedoch mindestens drei, noch bevorzugter mindestens vier oder sogar fünf der vorliegend beschriebenen Tests durchgeführt, wobei die jeweils relevanten Parameter bestimmt werden. Die Ergebnisse der einzelnen Tests werden insgesamt betrachtet, wobei eine geeignete Helminth-Präparation die vorgegebenen Grenzwerte in jedem Test erfüllen muß, damit hieraus der Schluß gezogen werden kann, dass die Helminth-Ei-Präparation für die pharmazeutische Anwendung geeignet ist.

Es besteht also ein großer Bedarf an einem industriell anwendbaren. Verfahren, das die biologische Aktivität von TSO und anderen Helminth-Eiern in verschiedenen Entwicklungsstadien umfassend und zuverlässig analysiert und dabei verschiedene biologische Funktionen der Helminth-Eier charakterisiert. Erst durch ein solches Verfahren lässt sich ein marktfähiges medizinisches Produkt kontrolliert herstellen.

Es ist eine Aufgabe der vorliegenden Erfindung, durch das im Folgenden näher ausgeführte Verfahren die biologische Aktivität von Helminth-Eiern umfassend zu analysieren und so einerseits eine enge Kontrolle des Herstellungsprozesses, andererseits eine sichere und therapeutisch effiziente Anwendung im Patienten zu ermöglichen.

Aus der komplexen Problemstellung und den Defiziten der bisher bekannten Verfahren wird deutlich, dass eine verlässliche Bestimmung der biologischen Aktivität in der Regel nicht durch einen einzelnen Verfahrensschritt erreicht werden kann.

Gegenstand der Erfindung ist daher ein Verfahren zur Bestimmung der biologischen Aktivität von Trichuris-Eiern, die vollständig embryonierte Larven enthalten und bei dem wenigstens drei der folgenden Bestimmungen durchgeführt werden:

Bestimmung der temperaturinduzierten Aktivität von Trichuris-Eiern mit biochemischen und/oder molekularbiologischen Methoden, insbesondere der Messung des ATP-Gehaltes, Bestimmung der Induzierbarkeit der Genexpression in embryonierten Trichuris-Eiern, mikroskopische Bestimmung der Motilität von im Ei befindlichen Trichuris-Larven über lange Beobachtungszeiträume nach Aktivierung durch Vorinkubation bei erhöhten Temperaturen, und/oder Bestimmung der Schlüpfrate von Trichuris-Larven im Versuchstier, wobei die aus dem Darminhalt zurückgewonnenen intakten embryonierten Eier im Vergleich zu einem internen Standard quantifiziert werden.

Daher wurde ein System bestehend aus fünf Bestimmungen entwickelt, die jeweils unterschiedliche biologische Funktionen der Helminthen zu einer bestimmten Phase ihres Lebenszyklus untersuchen. Zwar kann es während der einzelnen Herstellungsschritte einer pharmazeutischen Zubereitung ausreichend sein, nur eine Bestimmungsmethode anzuwenden, für die Bestimmung der biologischen Aktivität des Endprodukts müssen jedoch wenigstens 3 der nachfolgend in Tabelle 1 dargestellten Bestimmungen durchgeführt werden:

Tabelle 1

| Verfahrensschritt | Beispielhafte Parameter der biologischen Aktivität |
|---|---|
| 1. Bestimmung der Kopienzahl genomischer DNA | • Stadium der Larval-entwicklung zwischen L0 und L1<br>• Identität des Organismus |
| 2. Bestimmung der metabolischen Aktivität | • Viabilität der L1-Larve im Ei |
| 3. Untersuchung der induzierbaren Genexpression | • Aktivierbarkeit der ruhenden L1-Larve |
| 4. Bestimmung des Motilitätsindex | • Bewegungsfähigkeit der L1-Larve im Ei (Voraussetzung für das Schlüpfen) |
| 5. bestimmung der Schlüpfrate *in vivo* | • Schlüpfen der L1-Larve aus dem Ei (Voraussetzung für die Besiedlung des Wirtes) |

Für eine sichere Charakterisierung der biologischen Aktivität von pharmazeutisch verwendbaren Helminth-Eiern wird das komplette Verfahren mit wenigstens drei, bevorzugt 4 und noch bevorzugter allen 5 Schritten durchgeführt. Gegebenenfalls kann es für die Charakterisierung bestimmter Aspekte der biologischen Aktivität oder für die Überwachung von Teilschritten der Herstellung genügen, nur einzelne Schritte des Verfahrens durchzuführen.

Die fünf einzelnen Verfahrensschritte greifen auf Testprinzipien zurück, die in anderem Zusammenhang bereits beschrieben sind. Die Adaption auf embryonierte Helminth-Eier, insbesondere embryonierte Eier von *Trichuris suis,* ist neu und bedurfte der Entwicklung einer Reihe von neuen bisher noch nicht beschriebenen Teilschritten. Auch ist das gesamte Verfahren, das fünf unterschiedliche Aspekte der biologischen Aktivität prüft und so eine umfassende Charakterisierung ermöglicht, neuartig und für Helminth-Eier, insbesondere Eier von *Trichuris suis,* noch nicht zuvor beschrieben worden. Bevorzugtes gemeinsames Merkmal für drei der fünf beschriebenen Verfahren ist die Aktivierung von ruhenden *Trichuris* Larven durch Vorinkubation bei erhöhter Temperatur. Eine weitere Besonderheit dieses Verfahren liegt darin, dass es mit intakten, viablen Helminth-Eiern durchgeführt werden kann und, dass die gemessenen Parameter nur von der Aktivität der Helminth-Eier und nicht von Wirts-Faktoren oder den besonderen Fähigkeiten der den Test durchführenden Person abhängen, somit also objektiver als die bisher angewandten Verfahren sind. Hierdurch ist eine Standardisierung möglich, die für die pharmazeutische Verwendung erforderlich ist.

Durch das Verfahren ist es möglich, die biologische Aktivität von Helminth-Ei-Präparationen mit der für ein pharmazeutisches Produkt erforderlichen Genauigkeit zuverlässig zu bestimmen. Die genaue Bestimmung der biologischen Aktivität von Helminth-Eiern, wie sie in dieser Erfindung beschrieben wird, ist ein wesentlicher Schritt in der Herstellung eines anwendbaren pharmazeutischen Produktes.

Im folgenden werden die 5 Bestimmungsmethoden, die Bestandteil des Verfahrens sind, näher ausgeführt:

**a) Bestimmung der Kopienzahl genomischer DNA**

Bei jeder Zellteilung während der Embryonalentwicklung wird der Chromosomensatz verdoppelt und auf die beiden Tochterzellen verteilt. Daher kann die Kopienzahl der genomischen DNA im Ei als Korrelat für die Anzahl der Körperzellen der sich entwickelnden Larve angesehen werden. Die Bestimmung der Kopiezahl genomischer DNA kann also genutzt werden, um in einem beliebigen Stadium den Status der Embryonalentwicklung der sich entwickelnden Larven abzubilden. Dies ist ein klarer Vorteil gegenüber dem bisher beschriebenen Verfahren (Mikroskopische Bestimmung der Embryonierungsrate, s.o.), mit dem sich nur das Endergebnis der ca. 13 Wochen dauernden Embryonierung untersuchen lässt und die keinen Aufschluß darüber gibt, ob die Embryonalentwicklung tatsächlich vollständig abgeschlossen ist (d.h. ob die Zellzahl einer vollständig ausgebildeten L1-Larve entspricht). Die Embryonierung ist Teil des pharmazeutischen Herstellungsprozesses der Helminth-Eier. Mit der Bestimmung der Kopiezahl genomischer DNA ist erstmals eine prozess-begleitende Analytik möglich. Sie erlaubt zum einen eine rationale Entwicklung des Herstellungsprozesses und die Untersuchung des Einflusses von Prozess-Änderungen und zum anderen die routinemäßige Überwachung der Herstellung während der Embryonierungsphase.

Mit Hilfe der quantitativen PCR-Technologie kann eine Bestimmung der Kopiezahl einer bestimmten Nucleinsäuresequenz in einer Probe durchgeführt werden. Je mehr Kopien in einer bestimmten Nucleinsäuresequenz in der Probe vorhanden sind, umso schneller wird das Amplifikationsplateau erreicht. Durch Eichkurven kann die Anzahl der Kopien relativ genau bestimmt werden. Eine der hier üblicherweise verwendeten Methoden ist die Real Time PCR oder auch die sogenannte TaqMan PCR. Alternative Nucleinsäure-Amplifikationsmethoden sind dem Fachmann bekannt und können zur Bestimmung der Kopiezahl der genomischen DNA ebenfalls verwendet werden. Die genauen Kopienzahlen werden in der Regel mit geeigneten Eichkurven ermittelt.

Als bevorzugten Marker für die genomische DNA von Helminthen eignet sich bevorzugt die ITS1-5.8S-ITS2 Region, die für einen Teil der ribosomalen RNA kodiert. Für *Trichuris suis* (Cutillas et al., Parasitol Res 2001, 100: 383-389) sowie für andere *Trichuris*-Arten und weitere Helminthen ist die Sequenz der ITS1-5.8S-ITS2 Region bereits beschrieben. Auch ein Viabilitätstest, bei dem die Embryonalentwicklung von Ascaris suum mittels quantitativer PCR der IST-1 Region verfolgt wird, ist bereits beschrieben (Pecson et al., Appl.Envir.Microbiol., 2006, 72, 7864-7872).

Allerdings verfolgt dieser Test ein anderes Ziel, nämlich die Testung von Verfahren zur Inaktivierung von Helminthen.

Eine quantitative PCR (q-PCR), bei der eine Teilsequenz aus der ITS1-5.85-ITS2 Region amplifiziert wird, eignet sich besonders, um die Kopiezahl der Marker-Region und damit die Kopiezahl der genomischen DNA zu bestimmen. Da die ITS1 und ITS2 Elemente species-spezifisch sind, ist das Verfahren neben der Untersuchung des Stadiums der Embryonierung auch als qualitativer Identitätshachweis für den Organismus geeignet. Ein derartiger Test ist im Stand der Technik nicht beschrieben, zumal da bisher nicht bekannt war, dass das L1 Stadium von T.suis gemäß der Kopiezahl des Genoms etwa 1000 Zellen umfasst (vgl. Beispiel 1)).

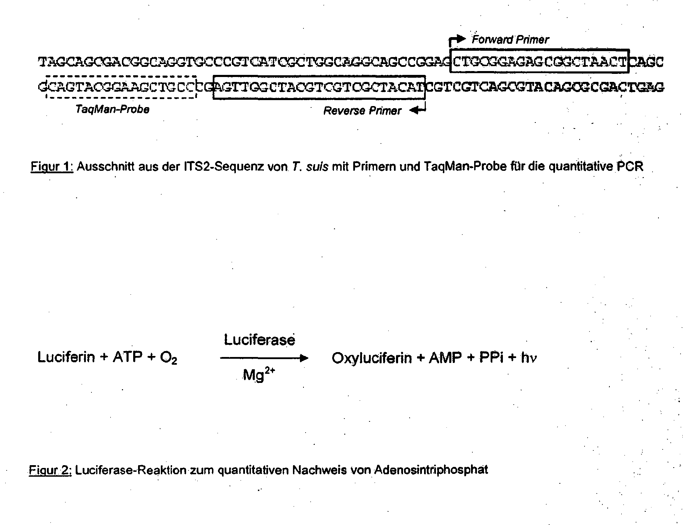

In Figur 1 ist die relevante Gensequenz von *Trichuris suis,* die beispielhaft für die Bestimmung der Kopiezahl genomischer DNA verwendet werden kann, dargestellt. SEQ ID NO:1 zeigt die relevante Gensequenz. Der Vorwärts-Primer ist als SEQ ID NO:2 wiedergegeben, der Rückwärts-Primer als SEQ ID NO:3 und die Sequenz der TaqMan-Probe ist als SEQ ID NO:4 wiedergegeben. Für den Fachmann ist offensichtlich, dass auch andere Teile aus dem Genom von *Trichuris suis* für die Bestimmung der Kopiezahl verwendet werden können. Voraussetzung für die Eignung der Sequenz ist, dass es sich hierbei um eine Nucleotidsequenz handelt, die spezifisch in *Trichuris suis* vorkommt und, dass es keine ähnlichen Sequenzen bei anderen Organismen gibt, die möglicherweise als Kontamination in die zu untersuchende Probe gelangen könnten.

Die Bestimmung der Kopiezahl der genomischen DNA hat auch einen Nebeneffekt. Es kann dadurch bestätigt werden, dass der gewünschte Organismus in der Präparation vorhanden ist und bei Verwendung geeigneter weiterer Sequenzen kann auch der Nachweis geführt werden, ob Kontaminationen mit anderen Organismen vorliegen.

Ein wesentlicher Aspekt, der bei der Bestimmung der Kopiezahl genomischer DNA nicht übersehen werden darf, ist, dass die Helminth-Eier vor der Messung aufgeschlossen werden müssen. Wie in den Beispielen gezeigt hat, sich hierbei ein "Potter-Homogenizer" als besonders geeignet herausgestellt, wobei in bevorzugter Ausführungsform ein Volumen von 2 ml eingesetzt wurde und eine Spaltbreite zwischen 0,01 und 0,03 mm eingestellt wurde. Die Homogenisierung wurder bevorzugt über einen Zeitraum von 5 bis 15 Minuten, bevorzugt etwa 10 Minuten durchgeführt. Der Zellaufschluß wird immer dann durchgeführt, wenn für den jeweiligen Untersuchungsschritt die Zellbestandteile in zugänglicher Form vorliegen müssen.

**b) Bestimmung der metabolischen Aktivität**

Viele Verfahren zur Bestimmung der Viabilität von Zellen basieren auf der Untersuchung der metabolischen Aktivität. Als Energieüberträger und -speicher spielt Adenosintriphosphat eine zentrale Rolle im Zellmetabolismus und kann damit als marker zur Bestimmung der intrazellulären Stoffwechselaktivität verwendet werden. Die vorliegende Erfindung zeigt, dass der Nachweis dieses Nukleotids auch für die Bestimmung der Viabilität von ausdifferenzierten Organismen wie L1-Larven von *Trichuris suis* verwendet werden kann.

Die Quantifizierung von Adenosintriphosphat in biologischen Systemen wird durch die Messung der Lumineszenz mit Hilfe der Luciferase-Reaktion ermöglicht. Die Lumineszenz ist definitionsgemäß die Emission "kalten" Lichts.

Lumineszenz-Systeme basieren auf der chemischen, biochemischen oder elektrochemischen Aktivierung von Substraten, die bei der Rückkehr in ihren Grundzustand einen Teil der Anregungsenergie in Form von Licht abgeben. Beim Nachweis von Adenosintriphosphat werden Luciferasen eingesetzt, die aus Leuchtkäfern (*Photinus pyralis*; *firefly*) isoliert wurden. Das eukaryontische Enzym katalysiert die Oxidation von Luciferin in Anwesenheit von Adenosintriphosphat, Sauerstoff und Magnesiumionen unter Lichtaussendung zu Oxyluciferin. Die Reaktionsgleichung ist in Figur 2 dargestellt.

Voraussetzungen für eine erfolgreiche Etablierung der Adenosintriphosphat-Bestimmung in embryonierten viablen Eiern von *Trichuris suis* sind die Stimulierung einer für die Lumineszenzmessung ausreichenden Adenosintriphosphat-Synthese in den L1-Larven, ein vollständiger Aufschluss der Eier durch ein geeignetes Homogenisierungsverfahren zur Freisetzung des intrazellulär gebildeten Nukleotids sowie die quantitative und damit störungsfreie Erfassung von Adenosintriphosphat aus der komplexen Matrix. Überraschend und bisher nicht beschrieben wurde gefunden, dass der ATP-Gehalt von L1-Larven vor Abschluss der Embryonalentwicklung niedrig ist und nur nach geeigneter Aktivierung durch Inkubation über einen längeren Zeitraum in einer bestimmten Temperatur auf einen konstant hohen Level angehoben wird. Weiterhin ist für die Differenzierung von lebenden und toten L1-Larven ein effektives Inaktivierungsverfahren erforderlich, so dass der Basiswert von Adenosintriphosphat in abgetöteten Organismen reproduzierbar bestimmt werden kann.

Die Bestimmung des ATP-Gehaltes zur Untersuchung der metabolischen Aktivität von Helminth-Ei-Populationen ist erfindungsgemäß bevorzugt.

Zur Untersuchung der metabolischen Aktivität einzelner Helminth-Eier, sind auch Färbemethoden mit Tetrazoliumverbindungen geeignet, die ursprünglich für frei zugängliche tierische Zellen in Zellkulturen oder histologische Schnitte entwickelt wurden. Die Tetrazoliumverbindungen werden in metabolisch aktiven Zellen durch die Wirkung mitochondrialer Enzyme zu farbigen Formazanen reduziert, die sich in den Zellen ablagern. Entscheidend für die Übertragung der Färbemethoden von frei zugänglichen tierischen Zellen auf die in Eiern befindlichen vielzelligen L1-Larven von Helminthen ist eine Vorbehandlung der Eier, die eine Permeation des Substrates erlaubt ohne die Viabilität der sich im Ei befindenden Larve zu beeinträchtigen. Die L1-Larven von *Trichuris suis* sind von einer rigiden Schale umgeben, die den Stoffaustausch mit der Umgebung stark einschränkt und die Permeation der für die Untersuchungen notwendigen Substanzen verhindert.

Als vorteilhaft für den schonenden Abbau der Ei-Hülle hat sich die Behandlung der Eier mit hypochloriger Säure erwiesen, die schon zuvor beschrieben worden ist (Beer, Parasitol., 1973., 67: 263-278) und die gegebenenfalls durch einen nachfolgenden enzymatischen Verdau mit Chitinase und Protease unterstützt werden kann.

Um die metabolische Aktivität bestimmen zu können, muß ein "Nullwert" ermittelt werden. Hierbei handelt es sich um einen Vergleichswert, von dem aus die jeweilige metabolische Aktivität ermittelt wird. In bevorzugter Ausführungsform wird der "Nullwert" mit inaktiven Helminth-Eiern ermittelt.

Als geeignetes Verfahren zur Inaktivierung der Eier hat sich die Kryoinaktivierung (Schockgefrieren und Lagerung bei -80°C über 24 Stunden) herausgestellt. Das Adenosintriphosphat-Signal von auf diese Weise inaktivierten Proben konnte nahezu auf Hintergrundrauschen reduziert werden. Die Herstellung kryoinaktivierter Ei-Proben erfolgt in Phosphat-gepufferter physiologischer Kochsalzlösung. Die Kryoinaktivierung kann selbstverständlich auch bei den anderen Bestimmungsverfahren eingesetzt werden.

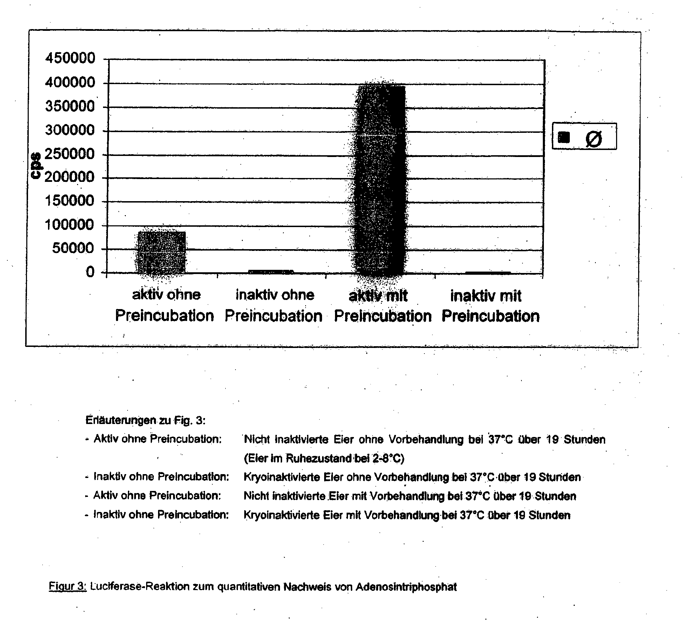

Figur 3 zeigt beispielhaft die Wirkung der Kryoinaktivierung beim quantitativen Nachweis von Adenosintriphosphat mit Hilfe der Luciferase-Reaktion. Durch die Kryoinaktivierung kann spezifisch die Wirkung der Präinkubation gemessen werden. Bei inaktivierten (abgetöteten) Helminth-Eiern ist auch nach Präinkubation kein Anstieg von Adenosintriphosphat erkennbar. Biologisch aktive Helminth-Eier weisen dagegen eine Aktivität auf, die durch den Anstieg des Adenosintriphosphats nach einer Vorbehandlung bei 37°C über eine Zeitdauer von 19 Stunden erzielt wird.

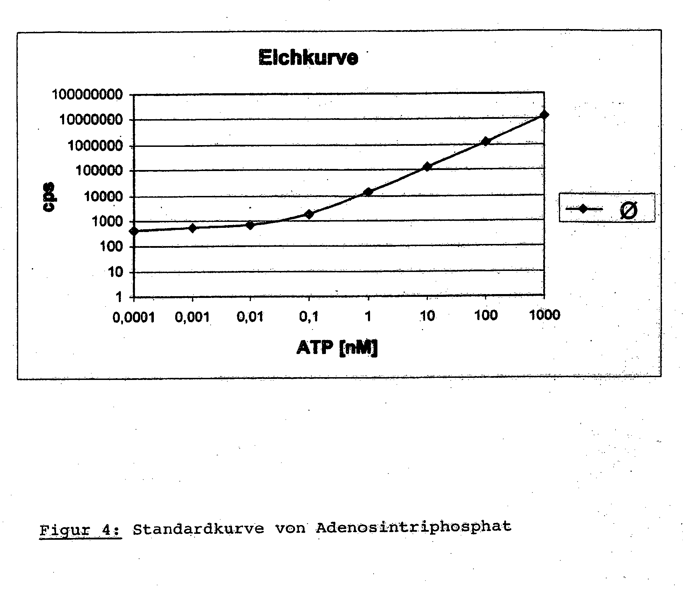

In einer bevorzugten Ausführungsform werden für jeden Ansatz 5500 Eier in 550 $\mu$l Lösung verwendet. Nach der Vorinkubation werden die Eier durch Zentrifugation abgetrennt (5 min bei 500 UpM) und in 225 $\mu$l Lysepuffer resuspendiert. Zur Freisetzung des Zellinhalts mit dem durch Inkubation gebildeten Adenosintriphosphats werden die Eier mit einem Potter-Homogenizer (Volumen 2 ml; Spaltbreite 0.01-0.03 mm) für 10 min homogenisiert. Nur dieses Verfahren führt zu einem vollständigen Aufschluss der Eier und damit zu einer quantitativen Freisetzung des Nukleotids. Um Adenosintriphosphatspaltende Hydrolasen zu inaktivieren, erfolgt der Aufschluss der Eier mit einem Lysepuffer (Bestandteil: Phosphorsäure pH 2). Von den einzelnen Ansätzen werden jeweils 50 $\mu$l auf 96-Mikrowellplatten auspipettiert und jeder Ansatz mit weiteren 100 $\mu$l Phosphatpuffer verdünnt. Durch Zugabe von 100 $\mu$l des kommerziell erhältlichen Adenosintriphosphat-Kits wird die Luciferase-Reaktion gestartet. Nach einer Inkubation von 2 Minuten wird die Mikrowellplatte im Luminometer ausgelesen und die Lumineszenz bestimmt (Fig. 3). Die Quantifizierung erfolgt gegen eine Standardkurve von Adenosintriphosphat in Phosphatpuffer (1.0 $\mu$M, 0.1 $\mu$M, 10 nM, 1 nM, 0,1 nM, 10 pM, 1,0 pM, 0,1 pM). Eine typische ATP-Eichkurve ist in Figur 4 dargestellt.

Durch gezieltes Aufstocken von Adenosintriphosphat zu homogenisierten kryoninaktivierten Proben von embryonierten Eiern konnte innerhalb des linearen Bereichs der Standardkurve (1 bis 1000 nM) eine Wiederfindung von 84% erzielt werden. Eine relevante Wechselwirkung der komplexen Matrix des Ei-Homogenats mit der Luciferasereaktion und der Lumineszenz ist damit nicht zu erkennen.

Das Beispiel zeigt eindeutig, dass die Lumineszenzmessung eingesetzt werden kann, um viable embryonierte Eier von *Trichuris suis* von inaktivierten und damit nicht viablen Eiern zu differenzieren. Die erfolgreiche Durchführung des Tests gelingt jedoch nur mit einer Kombination von optimierten Verfahrensparametern bestehend aus Proben-Aktivierung, Proben-Homogenisierung und der Herstellung geeigneter Kontrollen durch ein optimiertes Inaktivierungsverfahren. Der geeignete ATP-Gehält biologisch aktiver Helminth-Eier liegt in einem Bereich von mindestens 0,01 pmol ATP pro Ei.

**c) Untersuchung der induzierbaren Genexpression**

Das Prinzip dieses Verfahrens liegt in der Induktion der Gen-Expression, die nur in lebenden Zellen stattfinden kann und mit deren Hilfe zwischen lebenden und toten Larven differenziert werden kann. Zudem ist die Induzierbarkeit der Genexpression vermutlich Voraussetzung dafür, dass die ruhende L1-Larve verschiedene Aktivierungszustände durchlaufen kann, die notwendig sind, die nächsten Stadien des Lebenszyklus (Schlüpfen, Etablierung in der Mucosa etc.) zu induzieren.

Als besonders vorteilhaft erscheint die Untersuchung der Expression von Hitzeschock-Proteinen, da sich diese in einfacher Weise durch Temperatur-Erhöhung induzieren lassen und die gebildete Messenger-RNA (mRNA) im allgemeinen stabil gegen einen schnellen Abbau ist. Im Rahmen der vorliegenden Erfindung gelang in *Trichuris suis* erstmals der Nachweis einer Gen-Sequenz, die weitgehend homolog zum HitzeschockProtein hsp70 aus dem Helminthen *Caenorabhditis elegans* ist.

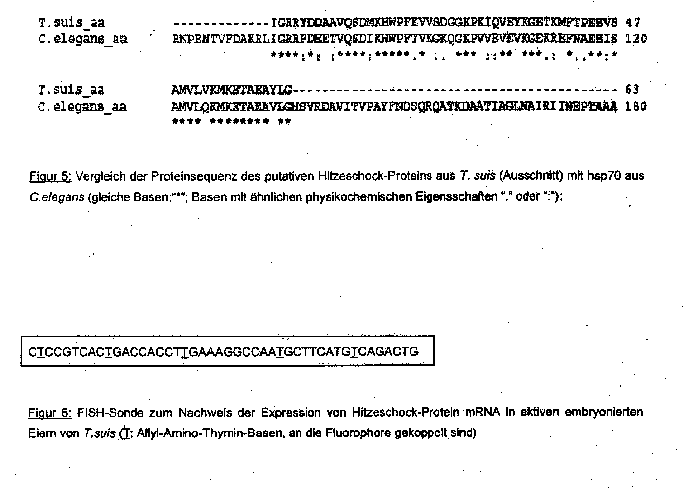

In einer bevorzugten Ausführungsform der vorliegenden Erfindung wird die Genexpression des Hitzeschockproteins von *T.suis* analysiert. In Figur 5 ist die Proteinsequenz von *Trichuris suis* (SEQ ID NO:5), dargestellt im Vergleich mit der Aminosäuresequenz des homologen Proteins aus *Caenorabhditis elegans* (SEQ ID NO:6).

Die induzierbare Genexpression kann durch Bestimmung des Gehalts an für ein bestimmtes induzierbares Protein kodierende Messenger-RNA erfolgen. Aus der Aminosäuresequenz kann die Nucleotidsequenz der Messenger-RNA abgeleitet werden. Für den Fachmann stellt es kein Problem dar, geeignete Vorwärts- und Rückwärts-Primer festzulegen und dazwischen die Sequenz einer TaqMan-Probe festzusetzen. Mit Hilfe einer Real Time PCR kann dann die Induktion des Gens bestimmt werden. Voraussetzung für eine aussagekräftige Bestimmung ist, dass auch eine Kontrollprobe gemessen wird, die abgetötete Wurmeier enthält. Außerdem muss es sich um ein Protein handeln, das einfach und verlässlich induzierbar ist. Eines der Beispiele ist das Hitzeschockprotein, es können jedoch ebenso gut auch andere durch bestimmte Reize induzierbare Gene für die Bestimmung herangezogen werden.

Der Nachweis der exprimierten Messenger-RNA auf Ebene einzelner Eier gelingt auch durch Fluoreszenz-in situ-Hybridisierung (FISH) mit einer geeigneten Gen-Sonde. Mit besonderem Vorteil lässt sich die Ei-Population anschließend mithilfe Durchflußcytometrie schnell und zuverlässig untersuchen.

Die Sequenz einer geeigneten Sonde zur in situ-Hybridisierung ist in Figur 6 (SEQ ID NO:7) dargestellt.

**d) Bestimmung der Motilität von Larven in intakten Helminth-Eiern**

Das Prinzip des Verfahrens beruht auf dem Nachweis von Körperbewegungen als Parameter für die Funktionalität der L1-Larve im Ei. Die Motilität der Larve ist Voraussetzung dafür, dass sie unter passenden Umgebungsbedingungen schlüpfen kann. Bei frei lebenden Larven und Würmern wie *C.elegans* gilt die Motilitäts-Untersuchung als zuverlässiger Viabilitäts- und Funktionsnachweis.

Im Rahmen der vorliegenden Erfindung wurde überraschend und erstmals am Beispiel von *Trichuris suis* gefunden, dass sich auch bei nicht frei lebenden, im Ei befindlichen Helminth-Larven durch exakte Einstellung der Umgebungstemperatur subtile Bewegungen induzieren lassen. Bisher beschrieben ist, dass die Larven nach Abschluss der Embryonierung in einen Ruhezustand verfallen und erst nach Aufnahme durch den passenden Wirt aktiviert werden und schlüpfen. Die Bewegungen der Larven im Ei sind sehr langsam und nur detektierbar, wenn die Eier wie in der vorliegenden Erfindung offenbart im Zeitraffer über einen langen Zeitraum mikroskopiert werden. In Kombination mit einer Bildanalyse-Software lässt sich der mikroskopische Motilitätstest automatisieren. Der Motilitätsindex als Parameter für die biologische Aktivität der Eier berechnet sich wie folgt:

$$\text{Motilitäts} - \text{Index} = \frac{\text{Anzahl der motilen Larven im Beobachtungsfeld}}{\text{Anzahl der untersuchten Eier im Beobachtungsfeld}}$$

Bei der Bestimmung der Motilität von Larven ist eine exakte Temperierung für den Erfolg der Methode entscheidend. Zunächst werden die Eier über einen Zeitraum von 2 bis 30 Stunden, bevorzugt 4 bis 20 Stunden bei einer exakt eingestellten Temperatur, die zwischen 36°C und 42°C, bevorzugt 37°C bis 41 °C und besonders bevorzugt bei 39,5°C liegt, vorinkubiert. Nach dieser Vorinkubation werden die Larven in ein geeignetes Mikroskop verbracht, wobei die eingestellte Temperatur zumindest annähernd beibehalten werden kann. Die Beobachtung erfolgt dann mit einer Zeitrafferaufnahme über einen Zeitraum von 2 Minuten bis 4 Stunden, bevorzugt 30 Minuten bis 2 Stunden bei einer Temperatur zwischen 36°C und 42°C, bevorzugt 38°C bis 40°C.

Figur 7 zeigt die Ergebnisse eines erfindungsgemäß bestimmten Motilitätsindex, wobei bei inaktivierten Wurmeiern keinerlei Motilität beobachtet werden kann, bei den aktiven Wurmeiern steigt dagegen der Motilitätsindex mit der Zeit an.

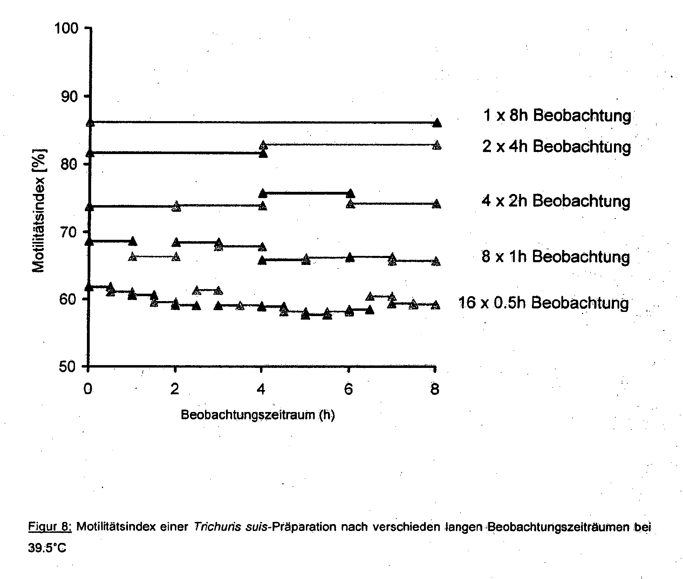

Figur 8 zeigt den Motilitätsindex im Zusammenhang mit verschiedenen Beobachtungszeiträumen.

### e) Bestimmung der Schlüpfrate im Intestinum

Das Prinzip dieses Verfahrens-Schritts besteht in der Bestimmung der Schlüpfrate von Helminth-Larven im Intestinum von Versuchtieren oder alternativ im Darminhalt, der zuvor aus Versuchstieren entnommen wurde.

Die Schlüpfrate kann in einer Ausführungsform in Darminhalt bestimmt werden, der Versuchstieren entnommen wird, und zwar bevorzugt am Beginn des Colons bzw. Ende des Duodenums. Die Schlüpfrate kann dann in dem Darminhalt bestimmt werden, ohne dass die Schlüpfrate direkt in einem Versuchstier bestimmt werden muss. Weiterhin lässt sich das Verfahren auch durch Untersuchung der Eier durchführen, die den Darm passiert haben und aus dem Kot zurückgewonnen werden. Dies hat den Vorteil, dass das Versuchstier nicht getötet werden muß und mehrfach zur Bestimmung der Schlüpfrate eingesetzt werden kann.

In dem neu entwickelten Verfahrensschritt wird in relativ kurzem Abstand nach Inokulierung der Darminhalt analysiert und die intakten Eier sowie die nach dem Schlüpfen zurückgelassenen Ei-Hüllen gezählt. Wesentlicher Vorteil gegenüber der im Stand der Technik beschriebenen Bestimmung der Infektivität ist, dass mit dem Schlüpfen nur die erste Phase des Lebenszyklus untersucht wird, die von individuellen Wirtsfaktoren nur wenig beeinflusst wird.

Überraschend konnte für *Trichuris suis* erstmals gezeigt werden, dass die Larven nicht nur im passenden Wirt, dem Schwein, sondern auch im Kaninchen quantitativ schlüpfen. Damit steht beispielsweise für *Trichuris suis* ein Versuchstier mit geringeren intestinalen Dimensionen zur Verfügung, was die Wiederfindung der mikroskopisch kleinen Eier und Ei-Hüllen deutlich erleichtert.

Überraschend wurde gefunden, dass sich mit kommerziellen für die Markierung von Proteinen entwickelten Fluoreszenzfarbstoffen die Ei-Schale dauerhaft anfärben lässt. Die Kopplung von Fluoreszenzfarbstoffen an die Ei-Hülle erleichtert wesentlich die Wiederfindung der Eier, da der Darminhalt fluoreszenzmikroskopisch untersucht werden kann.

Eine weitere wesentliche Neuerung ist der Einsatz von nichtembryonierten oder inaktivierten Helminth-Eiern als interner, unveränderlicher Standard. Diese haben die gleiche Transit- oder Verweilzeit im Darm, bleiben jedoch im Unterschied zu den embryonierten und biologisch aktiven Eiern bei der Darmpassage intakt. Durch Markierung der als interner Standard vorgesehenen Eiern mit einem zweiten (unterschiedlichen) Fluoreszenzfarbstoff kann leicht zwischen dem internen Standard und den zu untersuchenden Eiern unterschieden werden.

Zur Analyse werden im Darminhalt zu einem geeigneten Zeitpunkt nach Inokulierung die Anzahl der mit Fluoreszenzfarbstoff 1 markierten intakten Eier und leeren Ei-Hüllen der zu untersuchenden Probe sowie die mit Fluoreszenzfarbstoff 2 markierten intakten Eier des internen Standards untersucht. Die Schlüpfrate im Intestinum lässt sich wie folgt auf Basis der intakten (nicht geschlüpften) Eier berechnen.

Formel zur Berechnung der Schlüpf-Rate:

$$Schlüpf - Rate = 1 - \frac{\frac{[IE]i}{[IS]i}}{\frac{[IE]0}{[IS]0}}$$

In der nachfolgenden Tabelle 2 sind die PCR-Reaktion sowie die bevorzugten Bestimmungen und die damit bevorzugt erzielbaren Werte zusammengefasst, die bevorzugte Grenzwerte darstellen, ob die untersuchte Präparation die erforderliche biologische Aktivität aufweist.

Tabelle 2

| bevorzugte Bestimmung | Parameter | Zielgröße |
|---|---|---|
| a) PCR, genomische DNA | Kopienzahl genomischer DNA/Ei | Bereich: 50-1200 Kopien/Ei an d28 (Zu Beginn der Embryonierung liegt die Kopienzahl bei 1 Kopie/Ei; nach 28 Tagen Embryonierung sollte mindestens eine Kopienzahl von 50/Ei erreicht sein.) |
| b1) Metabolische Aktivität Variante 1: ATP Gehalt | Mittlerer ATP-Gehalt/Ei | Bereich: > 1 pmol ATP/embroyoniertes Ei |
| b2) Metabolische Aktivität Variante 2: Aktivitätsfärbung | Anteil der Eier mit positiver Aktivitätsfärbung | Bereich: ≥ 50 % (min. 50 % der Eier sollten eine positive metabolische Aktivitäts-färbung aufweisen) |
| c) Induzierbare Genexpression | Anteil der Eier, die eine induzierbare Genexpression zeigen | Bereich: ≥ 50 % (bei min. 50 % der Eier sollte sich die Genexpression induzieren lassen) |
| d) Motilitätsindex | Anteil der Eier, die eine motile Larve enthalten | Bereich: ≥ 50 % (min. 50 % der Eier sollten eine motile Larve enthalten) |
| e) Schlüpfrate | Anteil der Eier, die im Intestinum schlüpfen | Bereich ≥ 40 % (aus min. 40 % der verabreichten embryonierten Eier sollte eine Larve schlüpfen) |

In einer bevorzugten Ausführungsform umfasst das neue Testverfahren die Bestimmung der temperatur-induzierten Aktivierbarkeit von embryonierten Helminth-Eiern *in vitro.* Es werden zwei sich ergänzende Parameter gemessen (Motilitätsindex und mittlerer ATP-Gehalt) die zusammen den *in vitro*-activity score bilden. Der *in vitro* activity score zeigt eine gute Korrelation zur biologischen Aktivität in vivo. Er ist jedoch nur dann prediktiv für die biologische Aktivität, wenn die eingesetzten Larven vollständig embryoniert sind. Hierzu kommt eine quantitative PCR-Methode zum Einsatz, die die Kopienzahl des T.suis-Genoms im Ei mißt. Durch einen parallel durchgeführten neuartigen *in vivo*-Hatching-Test wird abgesichert, dass die geprüften Eier unter physiologischen Bedingung tatsächlich aktiv sind. Schematisch stellt sich das bevorzugte Verfahren dar, wie folgt:

```
                    ┌─────────────────┐
                    │   Embryonierte  │
                    │       TSO       │
                    └────────┬────────┘
                             │
                    ┌────────▼────────┐
                    │    ITS2 PCR     │
                    │  Zellzahl/Larve │
                    └────────┬────────┘
              ┌──────────────┴──────────────┐
        ┌─────▼──────┐              ┌────────▼──────┐
        │   ≥ 1000   │              │    <1000      │
        │(L1-Stadium)│              │   (unreif)    │
        └─────┬──────┘              └───────────────┘
  Aktivierung │
   ┌──────────┴──────────┐        ┌────────────────┐
   │  ┌──────┐ ┌────────┐│        │    In vivo     │
   │  │ ATP- │ │Motility││        │   Schlüpfrate  │
   │  │Gehalt│ │ Index  ││        └────────┬───────┘
   │  └──────┘ └────────┘│         ┌────────┴────────┐
   │   ┌──────────────┐  │   ┌─────▼─────┐  ┌────────▼────────┐
   │   │   in vitro   │  │   │  ≥ 50%    │  │    < 50%        │
   │   │activity score│  │   │(biologisch│  │  (biologisch    │
   │   └──────────────┘  │   │  aktiv)   │  │   inaktiv)      │
   └──────────┬──────────┘   └─────┬─────┘  └─────────────────┘
              └────────────┬───────┘
                  ┌────────▼──────────┐
                  │ Biologisch aktive TSO │
                  │ mit bekannter Potency │
                  └───────────────────┘
```

Ein wichtiger Aspekt der vorliegenden Erfindung ist die Bestimmung der Temperatur-induzierten Aktivierbarkeit in vitro.

L1-TSO gehen nach Abschluss der Embryonierung in einen Ruhezustand über, in dem Sie Jahre überdauern können, bis sie nach Aufnahme durch den Wirt aktiviert werden und schlüpfen. Unerwartet und bisher nicht beschrieben, wurde gefunden, dass die Eier auch in vitro lediglich durch eine Inkubation über einen bestimmten Zeitraum in einem bestimmten Temperaturfenster ohne die Zugabe weiterer Faktoren aktiviert werden können. Der aktivierte Zustand der Eier wird durch Bestimmung zweier sich ergänzender Parameter quantifiziert: die Motilität der Larven innerhalb der intakten Eier sowie die Bestimmung des ATP-Gehaltes von Lysaten zuvor aktivierter Eier. Unerwartet und im Gegensatz zu dem in der Literatur beschrieben Verhalten, induziert die hier beschriebene Vorinkubation subtile Bewegungen der Larve im Ei. Die Bewegung der Larven dauert über Stunden an, ohne dass die Larven absterben oder aus dem Ei schlüpfen. Daher ist eine bevorzugte Ausführungsform des hier dargestellten Verfahrens eine geeignete Vorinkubation der Eier, die zur Aktivierung führt. Über eine mikroskopische Beobachtung einer genügend großen Anzahl von Eiern im Zeitraffer gelingt es zuverlässig, den Anteil der Eier in der Population zu ermitteln, die eine aktive, motile Larve enthalten (Motilitäts-Index). Da diese Methode jedoch nicht zwischen verschiedenen Aktivitätszuständen der einzelnen Larven, also Abstufungen in der Motilität/Aktivität unterscheidet, wird sie mit der ATP-Bestimmung kombiniert, die ein quantitatives Maß für die Gesamtaktivität der untersuchten Ei-Population gibt. Beide Untersuchungsparameter, der Motilitätsindex als direkte, aber diskontinuierliche und der ATP-Gehalt als indirekte, aber kontinuierliche Größe ergänzen sich zu einer zuverlässigen Bestimmung der in vitro-Aktivität, die gut mit der in vivo-Aktivität korreliert. Aus beiden Parametern kann ein in vitro-Aktivitäts-Score berechnet werden, der gut mit der in vivo-Aktivität der Eier korreliert.

Die erfindungsgemäße in vitro-Methode unterscheidet sich deutlich von den bisher bekannten Verfahren. Die Bestimmung der Embryonierungsrate, bei die Eier lediglich optisch auf das Vorhandensein vollständig ausgebildeter Larven untersucht werden (Kringel et al.), lässt im Gegensatz zu dem hier dargestellten Verfahren keine Aussage über die Viabilität oder Aktivität der Larven zu. Die Bestimmung der Schlüprate in vitro durch Simulation der Magen und Darm-Passage erscheint im Vergleich zu der hier dargestellten Methode wesentlich komplexer und störanfälliger. Außerdem ist die Korrelation mit der in vivo-Aktivität für den in vitro Schlüpftest nicht belegt.

In der bevorzugten Ausführungsform der Erfindung wird der Embryonierungszustand bestimmt. Der *in vitro*-Aktivitäts-Score liefert nur dann sinnvolle, für die in vivo Situation prediktive, Ergebnisse, wenn die Larven die Embryonal-Entwicklung komplett durchlaufen haben. Unvollständig entwickelte Larven reagieren im Test auf temperaturinduzierte Aktivierbarkeit unter Umständen positiv, sind aber nicht funktional und wären nicht in der Lage im Wirt zu schlüpfen und dem normalen Lebenszyklus der Larve zu folgen. Eine vollständige Embryonalentwicklung wird bei den bisher bekannten Methoden mikroskopisch anhand von morphologischen Kriterien beurteilt. Diese Beurteilung bedarf großer Erfahrung, ist unsicher und kann naturgemäß nur an einzelnen Eiern erfolgen. Die erfindungsgemäße Methode misst dagegen objektiv die Kopienzahl des Genoms in einer großen Ei-Population, aus der sich die mittlere Anzahl somatischer Zellen pro Larve berechnen lässt.

Der Ergebnisse des Tests auf temperaturinduzierte Aktivierbarkeit werden bevorzugt durch einen neuartigen *in-vivo* Test abgesichert, der auf einfache und schnelle Weise zuverlässig die Schlüpfrate unter physiologischen Bedingungen bestimmt. Überraschend wurde gefunden, dass Larven aus biologisch aktiven Eiern im Intestinaltrakt quantitativ schlüpfen, während zuvor inaktivierte Eier die Darmpassage unverändert überstehen. In dem neu entwickelten Verfahren wird der Anteil der aktiven TSO in einer Präparation indirekt bestimmt, indem nur die inaktiven Eier gezählt werden, die den Intestinaltakt eines Versuchstiers unverändert passieren. Die Quantifizierung gelingt jedoch nur durch einen neu entwickelten internen Standard, der aus fluoreszenzmarkierten inaktiven Eiern besteht, der den Intestinaltrakt ebenfalls unverändert passiert. Überraschend und unerwartet wurde zudem gefunden, dass biologisch aktive TSO auch im Intestinum des Kaninchens quantitativ schlüpfen. Somit steht als Ersatz für das Schwein ein Tiermodell zur Verfügung, das kostengünstiger und einfacher zu halten ist und den Vorteil eines wesentlich kleineren intestinalen Volumens hat, was die Wiederfindung der mikroskopisch kleinen Eier erleichtert.

Der erfindungsgemäße *in vivo*-Test grenzt sich vom bekannten Infektivitätstest im Schwein ab. Die Bestimmung der Schlüpfrate erfordert nur 1-3 Tage, während der Infektivitätstest mehrere Wochen dauert. Zudem wird bei der Bestimmung der Schlüpfrate nur der erste Schritt des Lebenszyklus von *T.suis* im Wirt analysiert, der kaum von individuellen Wirtsfaktoren abhängig ist. Dagegen wird die Infektionsrate im Schwein vorzugsweise 3-4 Wochen nach Infektion bestimmt und unterliegt einer natürlichen biologischen Variabilität, die sich vor allem durch das individuell unterschiedlich ausgeprägte Immunsystem des Wirtes erklärt.

Ein weiterer Vorteil des hier dargestellten Assays besteht darin, das die Eier aus den Faeces der Tiere zurückgewonnen werden können und die Tiere deshalb für den Test nicht getötet werden müssen.

**Beispiele**

Beispiel 1:

Auf Basis der ITS2 Sequenz von *Trichuris suis* wurde mit Hilfe des TaqMan-Systems eine quantitative PCR-Methode entwickelt. Die Zielsequenz, die Primer und die TaqMan-Probe sind nachfolgend dargestellt. Die Zielsequenz wurde so gewählt, dass sie eine Differenzierung zwischen *Trichuris suis* und anderen *Trichuris*-Arten ermöglicht, deren Sequenz ebenfalls bekannt ist. So liefert der Test neben der Information über die ITS2-Kopienzahl auch den qualitativen Nachweis, dass es sich bei dem untersuchten Organismus tatsächlich um *Trichuris suis* handelt.

Zur Probenvorbereitung werden die Eier (1000 Eier in 500 µl Lösung) mit einem Potter-Homogenizer (Volumen 2ml; Spaltbreite 0.01-0.03 mm) für 10 min homogenisiert. Die mikroskopische Kontrolle belegt, dass sich überraschenderweise mit dieser Methode die Ei-Hüllen vollständig aufbrechen lassen, während andere bei der Genisolierung übliche Aufschlußverfahren bei Eiern von *Trichuris suis* erfolglos bleiben. Nach Zusatz von 1.54 µg Fisch-Sperma-DNA wird das Ei-Homogenat mit einem kommerziellen Kit zur Genisolisierung (DNeasy-Blood and Tissue-Kit; Qiagen) nach Vorschrift des Herstellers umgesetzt. Mit dem DNA-Isolat (Gesamtvolumen 50 µl) wird nach dem in der folgenden Tabelle dargestellten Protokoll eine PCR-Reaktion durchgeführt.

Tab. 3: Protokoll der PCR-Reaktion für den Zielabschnitt aus der ITS2-Region aus *T. suis*

| Instrument: | AB7900HT (Applied Biosystems) |
|---|---|
| Enzyme: | TaqMan Gene Expression Master Mix (Applied Biosystems) |
| Primer, TaqMan-Probe: | s. Figur 1 |
| Finale Konzentration Primer: | 900 nM |
| Finale Konzentration TaqMan Probe: | 200 nM |
| Proben-Volumen: | 5 µl |
| Gesamtvolumen PCR-Reaktion: | 25 µl |

(fortgesetzt)

| Instrument: | AB7900HT (Applied Biosystems) |
|---|---|
| Enzyme: | TaqMan Gene Expression Master Mix (Applied Biosystems) |
| Temperatur-Programm: | 50°C / 2 min<br>95°C / 10 min<br>40 Zyklen [95°C / 9 sec; 60°C / min] |

Die Amplifikations-Effizienz der Methode liegt zwischen 92% und 98% und die Sensitivität ist groß genug, um die Kopienzahl der ITS2-Gene in einem einzelnen Ei bestimmen zu können.

Mit der PCR-Methode wurde die ITS2-Kopienzahl von nicht-embryonierten und vollständig embryonierten *Trichuris suis* Eiern untersucht. Die Ergebnisse sind in der folgenden Tabelle dargestellt.

Tab. 4: Quantitative PCR für die ITS2-Region in nicht-embryonierten und embryonierten Eiern von *Trichuris suis*

| Probe (jeweils 1000 Eier) | Ct-Wert | Rel. Kopienzahl ITS2 |
|---|---|---|
| Nicht-embryonierte TSO (L0) | 25,5 | 1 |
| Embryonierte TSO (L1) | 15,5 | 1024 |

(Ct-Wert (Cycle treshold): Anzahl der PCR-Zyklen, die nötig sind, um ein Fluoreszenzsignal zu erreichen, das sich signifikant von der Background-Fluoreszenz unterscheidet)

Die PCR-Analyse zeigt, dass die ITS2-Kopienzahl in embryonierten TSO etwa um den Faktor 1000 höher liegt als die ITS2-Kopienzahl in nicht-embryonierten TSO. Da das nicht-embryonierte Ei (L0) als einzelne Zelle betrachtet werden kann, bedeutet dies, dass die L1-Larve von *T.suis* ca. 1000 somatische Zellen besitzt. Für *T. suis* ist die Anzahl der somatischen Zellen in der L1-Larve bisher nicht bekannt; Für den verwandten Organismus *C.elegans* wurde wurde eine Größe von 959 somatischen Zellen gefunden, was gut mit den hier gefundenen ca. 1000 Zellen übereinstimmt.

Um zu prüfen, ob sich mit dem Testverfahren verschiedene Stadien der Larvalentwicklung untersuchen lassen, wurden TSO über 4 Wochen embryoniert und regelmäßig mit der ITS2-PCR untersucht (Tab. 5).

Tab. 5: Relative ITS-2 Kopienzahl in Abhängigkeit von der Dauer der Embryonierung

| Embryonierungsdauer | Ct (T) - Ct (0) | Rel. Kopienzahl ITS2 |
|---|---|---|
| 0 Tage (L0-Zustand) | 0 | 1 |
| 7 Tage | 2,52 | 6 |
| 14 Tage | 6,04 | 66 |
| 21 Tage | 7,37 | 165 |
| 28 Tage | 7,76 | 216 |
| 90 Tage (L1-Zustand) | 10,0 | 1024 |

Das Beispiel zeigt eindeutig, dass das Verfahren genutzt werden kann, um verschiedene Stadien der Larval-Entwicklung zwischen L0 und L1 anhand der Anzahl der somatischen Zellen zu charakterisieren. Somit eignet sich dieses Verfahren besonders um den Verlauf der Embryonierung prozess-begleitend zu analysieren. Als besonders bevorzugte Spezifikation bei der Herstellung einer Helminth-Ei-Präparation erscheint das Erreichen einer relativen Kopienzahl von 50 - 1200 innerhalb der ersten 28 Tage der Embryonierung.

Beispiel 2:

Für den Nachweis von Adenosintriphosphat stehen kommerziell erhältliche Kits zur Verfügung, die unter anderem die für die Reaktion erforderlichen Luciferase-Enzyme sowie das Substrat Luciferin und Magnesiumsalze enthalten. Die Stimulation der Adenosintriphosphat-Bildung durch Vorinkubation der embryonierten Eier ist zwingend erforderlich. Der Adenosintriphosphat-Gehalt von viablen embryonierten Eier im Ruhezustand erlaubt keine ausreichende Differenzierung zu toten Eiern (Figur 3). Dabei hat sich ein Verfahren als geeignet erwiesen, bei dem die Eier über einen Zeitraum von 19 Stunden bei 37°C inkubiert werden. Ein wesentlicher Vorteil des entwickelten Analysenverfahrens besteht darin, dass für diese Inkubation keine Probenvorbereitung erforderlich ist. Die vorliegenden Ei-Suspensionen können unabhängig von der qualitativen und quantitativen Zusammensetzung des Mediums direkt verwendet werden. Selbst stark saure Medien mit pH-Werten <2 können ohne Probenvorbereitung eingesetzt werden und haben auf die Stimulierung von Adenosintriphosphat in den Eiern keine negative Auswirkung. Durch die Inkubation wird eine eindeutige Unterscheidung zwischen lebenden und töten F1-Larven ermtiglicht (Fig. 3).

Beispiel 3:

Mit Hilfe von Primern, die aus der Sequenz des in *Caenorhabditis elegans* gefundenen Hitzeschock-Proteins hsp70 und homologen Sequenzen aus *Trichuris muris* und *Trichuris vulpis* abgeleitet wurden, ließ sich in einem RNA-Isolat aus *Trichuris suis* eine Gensequenz amplifizieren. Die neue Sequenz aus *Trichuris suis* weist, übersetzt ein die Protein-Sequenz, eine 65% Übereinstimmung mit der Proteinsequenz des hsp70-Proteins aus C.elegans auf (Fig. 5).

Für das putative Hitzeschock-Protein aus *T.suis* wurde eine quantitative RT-PCR-Methode entwickelt. Die Quantifizierung erfolgte im Vergleich zur konstitutiv exprimierten 18S-rRNA. Aktive embryonierte Eier von *T.suis* wurden einem Hitzeschock (20 min bei 45°C) unterzogen und die Expression des Hitzeschock-Proteins wurde mit der in unbehandelten aktiven *T.suis* verglichen.

Tab. 6: Relative Expression des Hitzeschock-Proteins in aktiven embryonierten *T.suis*-Eiern (L1-Larven) (2 unabhängige Experimente mit jeweils 3 Proben; Fehler: Standard-Abweichung):

|  | Experiment #1 | Experiment #2 |
|---|---|---|
| Kontrolle | 1.075 ± 0.072 | 1.040 ± 0.029 |
| *T.suis* 45°C, 20min | 1.690 ± 0.212 | 1.467 ± 0.049 |

Die relative Expressionsanalyse zeigt eindeutig, dass sich die Expression der hsp-70 ähnlichen mRNA in *T.suis* durch Hitzeschock induzieren lässt (Tab. 6). Somit steht für den Aktivitätstest ein Gen zur Verfügung, dessen Expression sich durch Wahl geeigneter Versuchsbedingungen aktiv induzieren lässt.

Der Nachweis der aktiven Genexpression am einzelnen Ei gelingt mit Hilfe der Technik der Fluoreszenz-*in situ*-Hybridisierung (FISH). Um das putative Hitzeschock-Protein nachweisen zu können, wurde beipielsweise die in Figur 6 dargestellte Fluoreszenzmarkierte DNA-Sonde entwickelt (SEQ ID NO:7).

Nach Fluoreszenzmarkierung der aktiven Eier kann die Analyse der Ei-Population auch mittels der Durchfluß-Cytometrie untersucht werden. Gegenüber der mikroskopischen Analyse besteht der Vorteil darin, dass die Analyse schneller und objektiver erfolgt.

Das Beispiel zeigt eindeutig, dass sich mit dem hier beschriebenen Verfahren die Induktion der Genexpression in (einzelnen) Fielminth-Eiern untersuchen lässt. Damit steht erstmals ein Verfahren zur Verfügung, mit dem sich die Aktivierbarkeit ruhender L1-Larven von Helminthen untersuchen lässt.

Wird die Induktion der Genexpression als biologischer Aktivitätsnachweis bei der Herstellung von Helminth-Ei-Präparationen genutzt, so ist ein geeignetes Akzeptanzkriterium bei 25%-100% positiver Eier, bevorzugt 50 - 100 % positive Helminth-Eier.

Beispiel 4:

4.1 Abhängigkeit des Motilitäts-Index von den Untersuchungsbedingungen

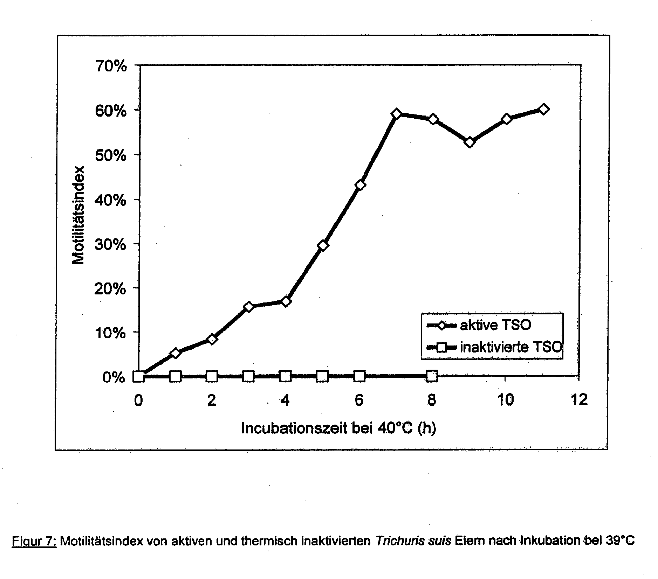

Aktive sowie durch Inkubation bei 48°C über 72 Stunden inaktivierte *Trichuris suis*-Eier wurden über 11 Stunden bei 39°C inkubiert. Stündlich wurden die Eier für jeweils 5 min beobachtet und der Motilitäts-Index bestimmt. Die Figur 7 zeigt die Entwicklung des Motilitäts-Index mit der Zeit.

Das Beispiel verdeutlicht, dass eine relative lange Vorinkubation bei erhöhten Temperaturen (hier: 7 Stunden bei 39°C) notwendig ist, um die Larven aus dem Ruhezustand zu aktivieren und einen konstant hohen Motilitäts-Index zu messen. Diese Beobachtung war unerwartet, da die fertig embryonierten im Ei befindlichen L1-Larven bisher als immobil galten (Parasitology 1973, 67: 253-262). Wahrscheinlich wurde die Motilität der Larven im Ei bisher nicht beobachtet, weil eine lange Vorinkubation notwendig ist und die langsamen Bewegungen nur im Zeitraffer sichtbar sind.

Der Vergleich von aktiven und inaktivierten Eiern zeigt eindeutig, dass sich durch Untersuchung der Motilität in einfacher Weise zwischen biologisch aktiven und inaktiven *Trichuris suis Eiern* differenzieren lässt.

Neben der Aktivierungsphase beeinflusst die Länge des Beobachtungsfensters den gemessenen Motilitätsindex, da die Larven sich nicht synchron bewegen und somit ein längeres Beobachtungsfenster die Wahrscheinlichkeit erhöht; in einem vitalen Ei die sporadischen Bewegungen zu erfassen. Dies zeigt die Untersuchung (Figur 7), bei der eine *Trichuris suis* Präparation nach Aktivierungsphase (8 Stunden bei 37°C) über verschieden lange Zeiträume bei 39.5°C bis 40°C beobachtet wurde. Die Untersuchung zeigt auch, dass die Motilität über die gesamte Beobachtungsperiode von 8 Stunden stabil bleibt.

Charakteristisches und neuartiges Merkmal dieses Verfahrens sind also die Aktivierungsphase mit exakter Temperaturkontrolle sowie das lange Beobachtungsfenster. Das Beispiel 4 zeigt, dass sich in ruhenden L1-Larven, die sich noch im Ei befinden, durch geeignete Temperatur- und Beoachtungs-Bedingungen Bewegungen induzieren und messen lassen. Mit dem Verfahren lässt sich auf einfache Weise die Motiliät als Vorrausetzung für das Schlüpfen und damit als Parameter für die biologische Aktivität der Eier bestimmen und eindeutig zwischen aktiven und inaktiven L1-Larven differenzieren.

4.2 Vorteilhafte Testbedingungen

Als vorteilhaft haben sich die folgenden Test-Bedingungen erwiesen: Eine Probe mit 15.000 Helminth-Eiern in 300 µl Phosphatpuffer, pH 7.4 wird in die Kammer einer 96-wellPlatte überführt (Bodenfläche: 0,31 cm$^2$). Bei dieser Einsaatdichte befinden bei 200-facher Vergrößerung ca. 80-150 Eier im Sichtfeld des Mikroskops. Nach einer 8-stündigen Inkubation bei 37°C (Aktivierungsphase) wird die Temperatur auf 39,5°C erhöht und es werden nacheinander 4 Beobachtungsfelder ausgewählt und für jeweils 2 Stunden beobachtet. Dabei wird ein Film mit 3 Bildern pro Minute aufgezeichnet. Anschließend wird die Anzahl der Eier sowie die Anzahl der sich bewegenden Larven bestimmt und der Motiliäts-Index berechnet. Der Motilitäts-Index der Probe ergibt sich aus dem Mittelwert der 4 Einzelmessungen.

4.3 Reproduzierbarkeit und Präzision - Vergleich mit dem Infektivitätstest

Zur Überprüfung der Reproduzierbarkeit des Motilitätstests unter den oben beschriebenen Bedingungen wurden 4 analoge Proben einer *Trichuris suis*-Charge an 4 unterschiedlichen Tagen untersucht. Die Messergebnisse sind in den folgenden Tabellen aufgeführt. Als Vergleich sind die Ergebnisse von 4 Messreihen mit dem Infektivitätstest aufgeführt, die mit der selben *Trichuris suis*-Charge erhalten wurden.

Tab. 7: Bestimmung des Motilitäts-Index einer *Trichuris suis-Charge* (Ergebnisse von 4 unabhängigen Untersuchungen bestehend aus jeweils 4 Messungen)

| Motilitätstest | | | | | Infektivitätstest | | | | |
|---|---|---|---|---|---|---|---|---|---|
| Messreihe | Messung | Motilitäts-Index (%) | | | Messreihe | Versuchstier | Infektivitätsrate (%) | | |
| | | Einzelmessung | Mittelwert | Präzision | | | Einzelmessung | Mittelwert | Präzision |
| 1 | #1 | 78,4 | 79,1 ± 2,1 | 2,7% | 1 | #1 | 27,4 | 25.9 ± 2.5 | 9,6% |
| | #2 | 77,5 | | | | #2 | | | |
| | #3 | 78,2 | | | | #3 | 23,5 | | |
| | #4 | 82,2 | | | | #4 | 29,5 | | |
| | | | | | | #5 | 24,5 | | |
| 2 | #1 | 73,0 | 79,6 ± 5,0 | 6,3% | 2 | #1 | 7,4 | 22.6 ± 13.2 | 58,4% |
| | #2 | 82,4 | | | | #2 | 29,9 | | |
| | #3 | 84,3 | | | | #3 | 26,7 | | |
| | #4 | 78,5 | | | | #4 #5 | 10,7 38,6 | | |
| 3 | #1 | 87,5 | 82,4 ± 5,1 | 6,2% | 3 | #1 | 17,1 | 32,2 ± 11,8 | 36,6% |
| | #2 | 85,4 | | | | #2 | 33,1 | | |
| | #3 | 80,8 | | | | #3 | 49,9 | | |
| | #4 | 76,0 | | | | #4 #5 | 31,8 29,1 | | |
| 4 | #1 | 81,1 | 79,8 ± 1,5 | 1,9% | 4 | #1 | 23,4 | 23,8 ± 2,9 | 12,1% |
| | #2 | 80,2 | | | | #2 | 23,7 | | |
| | #3 | 77,6 | | | | #3 | 27,9 | | |
| | #4 | 80,3 | | | | #4 | 24,1 | | |
| | | | | | | #5 | 19,8 | | |

Die Motilitäts-Indizes aus 4 unabhängigen Messreihen weichen um maximal 4,2% voneinander ab, dagegen liegt die maximale Abweichung der Infektivitätsrate aus 4 unabhängigen Messreihen bei 35,5%. Die Präzision der Einzelmessungen liegt beim Motilitätstest zwischen 1,9% und 6,3%, beim Infektivitätstest dagegen zwischen 9,6% und 58,4%. Der Motilitätstest ist dem Infektivitätstest also sowohl in der Reproduzierbarkeit als auch in der Präzision weit überlegen.

4.4 Korrelation zwischen Motilität und biologischer Aktivität (Richtigkeit) - Vergleich mit dem Infektivitätstest

Zur Überprüfung der Korrelation zwischen Motilität und biologischer Aktivität wurden Mischungen aus aktiven TSO und thermisch inaktivierten TSO hergestellt und mit dem Motilitätstest nach den in 4.2 beschriebenen Bedingungen untersucht. Die thermische Inaktivierung erfolgte durch Erhitzen der Eier bei 48°C über 72 Stunden. Um objektive Zählergebnisse zu erhalten, wurden die Proben vor der Messung verblindet.

Die relative biologische Aktivität wurde als Quotient aus dem gemessenen Motilitätsindex der Probe und dem zuvor bestimmten Motilitätsindex der aktiven TSO berechnet. Die Richtigkeit der Messung ergibt sich aus dem Vergleich relativen biologischen Aktivität und dem tatsächlichen Anteil der aktiven Eier in der Probe.

Eine analoge Messreihe mit TSO aus der gleichen Charge wurde mit dem Infektivitätstest durchgeführt. Die Versuchstiere wurden mit unterschiedlichen Mengen aktiver TSO mit bekannter Infektivitätsrate infiziert. Auf eine Zumischung von inaktivierten Eiern wurde verzichtet. Die Ergebnisse sind in den nächsten Tabellen dargestellt.

Tab. 8: Richtigkeit und Präzision des Motilitätstests

| Probe | 1 | 2. | 3 | 4 | 5 | 6 |
|---|---|---|---|---|---|---|
| Anteil aktive TSO in der Probe = Erwartungswert der rel. biol. Aktivität | 0% | 30,0% | 50% | 70% | 90,0% | 100% |
| Motilitätsindex, Messung #1 | 0% | 25,2% | 39,6% | 56,1% | 72,7% | 89.8% |
| Motilitätsindex, Messung #2 | 0% | 26,5% | 39,8% | 56,5% | 78,3% | 86,3% |
| Motilitätsindex, Messung #3 | 0% | 20,6% | 42,7% | 55,3% | 79,1% | 87,3% |
| Motilitätsindex, Messung #4 | 0% | 20,0% | 43,5% | 58,9% | 76,9% | 87,0% |
| Motilitätsindex, Mittelwert | 0% | 23,1% | 41,4% | 56,7% | 76,8% | 87,6% |
| Motilitätsindex, 100% aktive TSO | 80,2% | 80,2% | 80,2% | 80,2%, | 80,2% | 80,2% |
| Rel. Biologische Aktivität | 0% | 28,8% | 51,6% | 70,7% | 95,7% | 109,2% |
| Richtigkeit (Abweichung vom Erwartungswert) | ± 0% | - 4,1% | + 3,3% | + 1,0% | + 6,4% | + 9,2% |
| Präzision (Streuung der Messwerte) | n.a. | ± 14,2% | ± 4,8% | ± 2,8% | ± 3,7% | ± 1,7% |

Tab. 9: Richtigkeit und Präzision des Infektivitätstests

| Probe | 1 | 2 | 3 | 4 |
|---|---|---|---|---|
| Anteil aktive TSO in der Probe = Erwartungswert der rel. biol. Aktivität | 0% | 25,0% | 50,0% | 100,0% |
| Infektivitätsrate, Versuchstier#1 | 0% | 4,1% | 12,4% | 52,2% |
| Infektivitätsrate, Versuchstier #2 | 0% | 6,2% | 17,2% | 6,3% |
| Infektivitätsrate, Versuchstier #3 | 0% | 0,4% | 20,4% | 31,1% |
| Infektivitätsrate, Versuchstier #4 | 0% | 9,5% | 22,6% | 36,0% |
| Infektivitätsrate, Versuchstier #5 | | 2,3% | 11,8% | 37,9% |
| Infektivitätsrate, Mittelwert | 0% | 4,5% | 16,9% | 32,7% |
| Infektivitätsrate, 100% aktive TSO | 26,1% | 26,1% | 26,1% | 26,1% |
| Rel. Biologische Aktivität | 0% | 17,0% | 64,6% | 125,2% |
| Richtigkeit (Abweichung vom Erwartungswert) | ± 0% | - 31,8 % | + 29,1% | + 25,2% |
| Präzision (Streuung der Messwerte) | n.a. | ± 79,2% | ± 28,4% | ± 51,1% |

Mit dem hier dargestellten Motilitätstest lässt sich die relative biologische Aktivität einer TSO-Probe über den gesamten Bereich zwischen 0% und 100% mit einer Abweichung von < 10% vom Erwartungswert richtig bestimmen. Die Präzision der Messung (Streuung der Messwerte) ist im Bereich zwischen 50% und 100% relativer biologischer Aktivität kleiner als 5%. Bei Bestimmungen niedrigerer relativer biologischer Aktivitäten liegt die Präzision unter 15%.

Die Ergebnisse des Infektivitätstests weichen im Bereich zwischen 0% und 100% relativer biologischer Aktivität um ca. 25-32% vom Erwartungswert ab. Die Streuung der Messwerte liegt zwischen 28% und 80%.

Das Beispiel zeigt eindeutig, dass der Motilitätsindex linear mit der relativen biologischen Aktivität korreliert. Bezüglich der Richtigkeit und Präzision ist der Motilitätstest dem Infektivitättstest weit überlegen.

Ein geeigneter Bereich für den Motilitätsindex als Maß für die biologische Aktivität von Helminth-Ei-Präparationen liegt bei 30% - 100%, bevorzugt 60 - 100%.

Beispiel 5:

Beispielhaft wurden Kaninchen verschiedene Mischungen aus embryonierten, nichtembryonierten und inaktivierten, embryonierten Eiern oral verabreicht. Nach 8 Stunden wurde der Darminhalt der Versuchtiere mikroskopisch untersucht und die intakten Eiern sowie die leeren Eihüllen wurden ausgezählt. Da die Eier in diesem Versuch nicht fluoreszenzmarkiert waren, wurde die Schlüpfrate aus dem Verhältnis der intakten embryonierten und nicht-embryonierten Eiern berechnet (nach der Formel oben mit [IE] = Anzahl embryonierter Eier und [IS] = Anzahl nicht-embryonierter Eier). Die nachfolgende Tabelle gibt die Ergebnisse der Untersuchung sowie die daraus berechnete Schlüpf-Rate wieder:

Tab. 10: Anzahl der intakten nicht-embryonierten und embryonierten *Trichuris suis* Eier, sowie der leeren Ei-Hüllen im Darminhalt von Kaninchen 8h nach oraler Verabreichung von Mischungen embryonierter, nicht-embryonierter und inaktivierter Eier

| Gruppe | Inokulierungsdosis | | | Eier/Eihüllen im Darminhalt | | | Schlüpfrate |
|---|---|---|---|---|---|---|---|
| | EE | IEE | NEE | EE | NEE | ES | |
| #1 | 36.000 | | 44.000 | 538 | 2050 | 2681 | 67.9% |
| #2 | | 36.000 | 44.000 | 7525 | 8166 | 65 | -12,6% |
| #3 | | | 80.000 | | 3860 | 15 | n.a. |
| (EE: intakte embryonierte Eier; IEE: intakte hitze-inaktivierte embryonierte Eier; NEE: intakte nicht-embryonierte Eier; ES: leere Ei-Hüllen; n.a.: nicht anwendbar) | | | | | | | |

Der Vergleich der Daten aus den Gruppen 1-3 zeigt, dass nur aktive embryonierte Eier in der Lage sind, im Kaninchen zu schlüpfen (erkennbar durch die deutliche Abnahme der Anzahl intakter embryonierter Eier und der Anwesenheit einer großen Anzahl leerer Ei-Hüllen). Dagegen bleiben inaktivierte embryonierte Eier im Intestinum unverändert (Gruppe 2). Dies belegt eindeutig, dass man mit dem Verfahren zwischen biologisch aktiven und inaktivierten Eiern differenzieren kann. Zudem bleiben auch nichtembryonierte Eier bei der Darm-Passage intakt (Gruppe 3). Dies erlaubt eine Nutzung von nicht-embryonierten Eiern als interner Standard, der den Darm der Versuchstiere durchläuft ohne abgebaut zu werden. Dieser interne Standard ist notwendig, da die Wiederfindung der Eier im Darminhalt nur unvollständig ist und somit die absolute Anzahl der wiedergefundenen intakten Eier nicht aussagekräftig ist. Zur Berechnung der Schlüpfrate unter Berücksichtigung des internen Standards dient die oben angegebene Formel. Nach dieser Formel zeigen die hier untersuchten *Trichuris suis* Eier eine Schlüpfrate von 67.9%, während die inaktivierten *Trichuris suis* Eier eine Schlüpfrate von 0% (rechnerisch: -12.6%) zeigen.

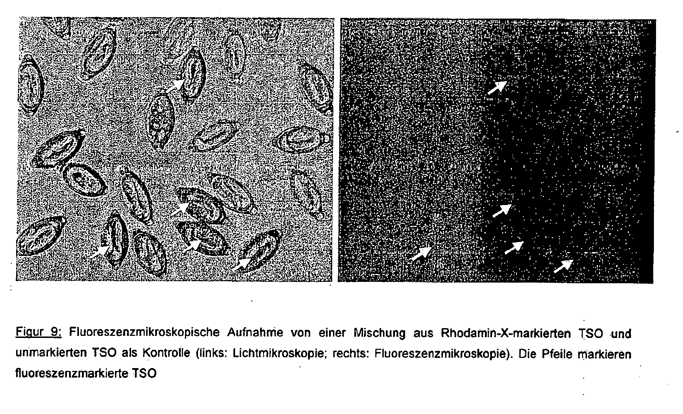

Um eine bessere Wiederfindung und eine klarere Differenzierung zwischen den Eiern der Probe und den Eiern des internen Standards zu erzielen, wurden *Tcichuris suis* Eier mit einer Fluoreszenz-Sonde kovalent markiert. Beispielhaft wurde als Fluoreszenzsonde Rhodamin X - succinimidyl - ester eingesetzt, das nach Angaben des Herstellers mit primären Aminogruppen in Proteinen reagieren kann. Eine Suspension von 1000 Trichuris suis Eiern in 200 $\mu$l Phosphatpuffer, pH 7.4, wurde mit 20 $\mu$l einer 1M Natriumbicarbonatlösung versetzt und anschließend mit 5 $\mu$l einer 0.5%-igen Lösung von Rhodamin X - succinimidyl - ester in DMSO umgesetzt. Die Lösung wurde unter Schwenken 1 h bei Raumtemperatur inkubiert. Anschließend wurden die Eier insgesamt 8 mal durch Zentrifugation (10 min bei 500 rpm) und Austausch des überstehenden Puffers gereinigt. Die Fluoreszenzmikroskopie zeigt eindeutig die Rotfärbung der Ei-Hülle (Fig. 9).

Die Fluoreszenzmarkierung lässt sich vorteilhaft zur mikroskopischen Auswertung der Schlüpfrate nutzen. Sie bietet darüber hinaus auch die Möglichkeit zur objektiven und schnellen Analyse mittels fluoreszenzaktivierter Durchflußzytometrie.

Insgesamt zeigt das Beispiel eindeutig, dass sich mit dem hier dargestellten Verfahren die die Phase des Schlüpfens quantitativ untersuchen und zur Bestimmung der biologischen Aktivität nutzen lässt. Ein geeigneter Bereich für die Schlüpfrate als Maß für die biologische Aktivität von Helminth-Ei-Präparationen liegt bei 25%-100%.

Beispiel 6:

Korrelation der temperaturinduzierten Aktivierbarkeit *in vitro* mit der biologischen Aktivität *in vivo*

Um die temperaturinduzierte Aktivierbarkeit mit der biologischen *in vivo* Aktivität zu korrelieren, wurden 12 TSO-Proben unterschiedlicher Qualität untersucht. Die Proben wurden zunächst mit der PCR-Methode auf die Vollständigkeit der Embryonalentwicklung untersucht. Anschließend wurde der Motilitätsindex und der ATP-Gehalt nach temperaturinduzeirter Aktivierung bestimmt. Parallel wurde für jede der 12 Proben ein Infektivitätstest im Schwein durchgeführt. Der Infektivitätstest wurde nach der bei Kringel et al. beschriebenen Methode in jeweils 5 Schweinen durchgeführt. Die beiden in vitro-Parameter Motilität und ATP-Gehalt wurde in einem in vitro activity score zusammengefasst:

$$In\ vitro\ activity\ score = (Motility\ Index + \frac{ATP - Gehalt}{50\ nM}) * 0.5$$

Tabelle 11: Bestimmung des Aktivitätsindex von mehreren Proben

| Probe | Zellzahl/ Larve (ITS2 PCR) | ATP-Gehalt [nmol] | Motility Index [%] | In vitro activity score [%] | Infektivität [%] |
|---|---|---|---|---|---|
| A | > 1000 | 6,13 ± 0,8 | 15,30% ± 5,9% | 13,8% ± 3,7% | 7,9% ± 11,5% |
| B | > 1000 | 13,40 ± 4,9 | 23,40% ± 4,0% | 25,1% ± 6,9% | 24,3% ± 1,6% |
| C | > 1000 | 20,70 ± 2,5 | 51,80% ± 3,2% | 46,6% ± 4,1% | 39,8% ± 3,7% |
| D | > 1000 | 36,35 ± 1,8 | 45,10% ± 1,2% | 58,9% ± 2,4% | 43,8% ± 6,7% |
| E | > 1000 | 32,75 ± 6,9 | 85,00% ± 2,8% | 75,3% ± 8,3% | 46,8% ± 10,3% |
| F | > 1000 | 35,73 ± 3,0 | 88,80% ± 1,1% | 80,1% ± 3,5% | 50,8% ± 11,5% |
| G | > 1000 | 41,27 ± 1,6 | 86,90% ± 3,5% | 84,7% ± 3,3% | 67,0% ± 5,0% |
| H | > 1000 | 40,20 ± 4,0 | 93,20% ± 1,5% | 86,8% ± 4,8% | 73,4% ± 9,2% |
| I | > 1000 | 51,88 ± 4,0 | 76,80% ± 1,2% | 90,3% ± 4,7% | 81,1% ± 4,5% |
| J | > 1000 | 46,60 ± 3,5 | 88,60% ± 2,4% | 90,9% ± 4,7% | 80,1% ± 4,4% |
| K | > 1000 | 46,44 ± 7,0 | 91.10% ± 1,1% | 92,0% ± 7,6% | 78,8% ± 6,2% |
| L | > 1000 | 48,67 ± 3,9 | 91,80% ± 2,3% | 94,6% ± 5,0% | 65,6% ± 17,8% |

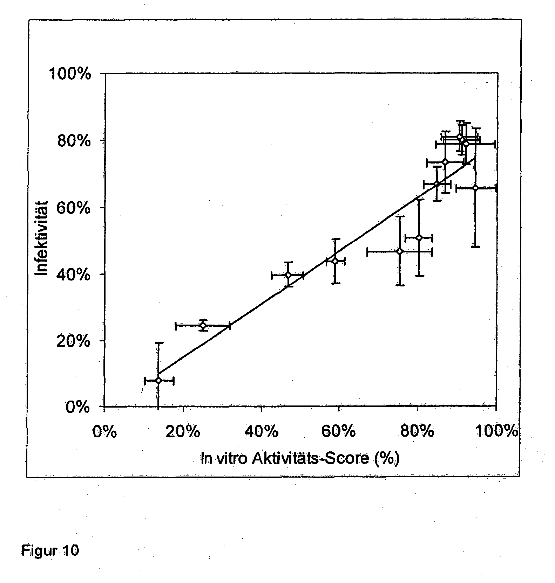

Die Korrelation zwischen in vitro activity score und Infektivität ist in der Figur 10 gezeigt.
Das Beispiel zeigt eine gute Korrelation des in vitro-Aktivitäts-Scores mit der biologischen Aktivität *in vivo*. Leichte Abweichungen bei einzelnen Proben sind wahrscheinlich eher auf Schwächen des in vivo-Tests zurückzuführen, der mit einer durch die biologische Variabilität (Wirtsfaktoren) bedingten instrinsischen Unsicherheit behaftet ist.

Die Messung einer Probe mit dem Infektivitätstest dauert 6 Wochen, erfordert den Einsatz von 5 Schweinen und ist mit erheblichem Arbeitsaufwand verbunden. Der hier vorgestellte in vitro-Test bestehend aus drei der fünf vorgestellten Verfahren dauert 1-2 Tage und lässt sich mit vergleichsweise geringem Arbeits- und Materialaufwand betreiben.

**Patentansprüche**

1. Verfahren zur mit der für ein pharmazeutisches Produkt erforderlichen Genauigkeit zuverlässigen Bestimmung der biologischen Aktivität von embryonierten *Trichuris* Eiern, bei der wenigstens 3 der nachfolgenden Bestimmungen durchgeführt werden:

    a) Bestimmung und/oder Bestätigung des Stadiums der Embryonalentwicklung von Helminth-Eiern mit Hilfe der quantitativen PCR-Analyse unter Verwendung geeigneter Marker-Sequenzen zur Ermittlung der Kopienzahl der genomischen DNA,
    b) Bestimmung der metabolischen Aktivität von embryonierten Helminth-Eiern mit biochemischen und/oder molekularbiologischen Methoden wobei zur Bestimmung der metabolischen Aktivität von embryonierten *Trichuris*-Eiern der ATP-Gehalt gemessen wird und/oder die *Trichuris-Eier* zunächst mit einem Vorbehandlungsmittel ausgewählt aus hypochloriger Säure, Chitinase und/oder Protease behandelt werden und dann mit Tetrazoliumsalzen angefärbt werden,
    c) Bestimmung der Induzierbarkeit der Genexpression in embryonierten Helminth-Eiern,
    d) mikroskopische Bestimmung der Motilität von im Ei befindlichen Helminth-Larven über lange Beobachtungszeiträume nach Vorinkubation bei erhöhten Temperaturen, und/oder

e) Bestimmung der Schlüpfrate von *Trichuris*-Larven im Versuchstier, wobei die aus dem Darminhalt zurückgewonnenen intakten embryonierten Eier im Vergleich zu einem internen Standard quantifiziert werden.

2. Verfahren gemäß Anspruch 1a), bei dem mittels quantitativer PCR-Analyse unter Verwendung geeigneter, für *Trichuris suis* spezifischer Sequenzen die Kopienzahl der genomischen DNA bestimmt wird.

3. Verfahren gemäß Anspruch 1b), **dadurch gekennzeichnet, dass** die *Trichuris-Eier* vor der Luminiszenzmessung unter folgenden Bedingungen vorinkubiert werden:

aa) zwischen 36°C und 42°C
bb) zwischen 2 und 30 Stunden
cc) in einem Suspensionsmedium mit einem pH-Wert zwischen 0.1 und 3.

4. Verfahren gemäß Anspruch 1c), **dadurch gekennzeichnet, dass** die Induzierbarkeit eines Hitzeschockproteins bestimmt wird.

5. Verfahren gemäß Anspruch 1c), **dadurch gekennzeichnet, dass** der Nachweis der Expression durch Hybridisierung mit einer fluoreszenzmarkierten Nucleotid-Sonde erfolgt.

6. Verfahren gemäß Anspruch 1c), **dadurch gekennzeichnet, dass** die Hybridisierung mit Hilfe der Durchflußzytometrie nachgewiesen wird.

7. Verfahren gemäß Anspruch 1d), **dadurch gekennzeichnet, dass** die Motilität der im Ei befindlichen Helminth-Larven mikroskopisch über Zeiträume von 2 min bis 8 Stunden mit Hilfe von Zeitrafferaufnahmen bestimmt wird.

8. Verfahren gemäß Anspruch 1e), **dadurch gekennzeichnet, dass** die zu überprüfenden Helminth-Eier mit Fluoreszenzsonden markiert werden und die internen Standards mit andersfarbigen Fluoreszenzsonden markiert werden.

9. Verfahren gemäß Anspruch 1e), **dadurch gekennzeichnet, dass** der Darminhalt von Kaninchen und/oder Schweinen als Prüfsystem verwendet wird.

10. Verfahren gemäß Anspruch 1e), **dadurch gekennzeichnet, dass** als interner Standard für die Ermittlung der Hatching-Rate nicht-embryonierte oder inaktivierte *Trichuris*-Eier verwendet werden.

11. Verfahren nach einem der vorhergehenden Ansprüche, **dadurch gekennzeichnet, dass** wenigstens vier Bestimmungen ausgewählt unter den Bestimmungen 1a), 1b), 1c), 1d) und/oder 1e) durchgeführt werden.

12. Verfahren nach einem der vorhergehenden Ansprüche, **dadurch gekennzeichnet, dass** die *Trichuris-Eier* vor der Durchführung der Bestimmung über einen Zeitraum von wenigstens 30 Minuten bis 24 Stunden unter genau standardisierten Bedingungen vorinkubiert wurden, wobei die Vorinkubation gegebenenfalls auch eine Änderung eines wesentlichen Parameters, insbesondere der Temperatur, beinhaltet.

## Claims

1. A method for reliably determining with the accuracy required for a pharmaceutical product the biological activity of embryonated *Trichuris* eggs in which at least three of the following determinations are carried out:

a) Determination and/or confirmation of a stage of embryonal development of helminth eggs with the aid of quantitative PCR analysis by using suitable marker sequences for ascertaining the copy number of genomic DNA,
b) Determination of metabolic activity of embryonated helminth eggs by means of biochemical and/or molecular biological methods wherein the ATP content is measured to determine the metabolic activity of embryonated *Trichuris* eggs and/or the *Trichuris* eggs are initially treated with a pre-treatment agent selected from hypochlorous acid, chitinase and/or protease and then stained with tetrazolium salts,
c) Determination of inducibility of gene expression in embryonated helminth eggs,
d) Microscopic determination of motility of herminth larvae in the egg over long observations periods after preincubation at increased temperatures, and/or

e) Determination of the hatching rate of *Trichuris* larvae in a laboratory animal wherein intact embryonated eggs recovered from contents of the animal's intestine are quantified compared to an internal standard.

2. The method according to claim 1a), in which by means of quantitative PCR analysis using specific sequences suitable for *Trichuris suis* the copy number of the genomic DNA is determined.

3. The method according to claim 1 b), wherein the *Trichuris* eggs are pre-incubated before luminescence measurement under the following conditions:

aa) between 36°C and 42°C
bb) between 2 and 30 hours
cc) in a suspension medium having a pH between 0.1 and 3.

4. The method according to claim 1c), wherein the inducibility of a heat shock protein is determined.

5. The method according to claim 1c), wherein the expression is detected by hybridization with a fluorescence-marked nucleotide probe.

6. The method according to claim 1c), wherein hybridization is detected with the aid of flow cytometry.

7. The method according to claim 1d), wherein the motility of the helminth larvae in the egg is determined microscopically over period of 2 minutes to 8 hours with the aid of time-lapse recordings.

8. The method according to claim 1e), wherein the helminth eggs to be examined are marked with fluorescence probes and the internal standards are marked with different coloured fluorescence probes.

9. The method according to claim 1e), wherein the contents of the intestine of rabbits and/or pigs are used as the test system.

10. The method according to claim 1e), wherein non-embryonated or inactivated *Trichuris* eggs are used as the internal standard for ascertaining the hatching rate.

11. The method according to any one of the preceding claims, wherein at least four determinations selected from among the determinations 1a), 1b), 1c), 1d) and/or 1e) are carried out.

12. The method according to any one of the preceding claims, wherein the *Trichuris* eggs were pre-incubated before carrying out the determination over a period of at least 30 minutes to 24 hours under precisely standardized conditions, wherein the pre-incubation, if required, also contains a change to an essential parameter, in particular the temperature.

**Revendications**

1. Procédé servant à déterminer, avec fiabilité et avec la précision qui s'impose pour un produit pharmaceutique, l'activité biologique d'oeufs de *Trichuris* embryonnés, où au moins 3 des déterminations qui suivent sont réalisées :

a) la détermination et/ou la confirmation du stade du développement embryonnaire d'oeufs d'helminthes à l'aide de l'analyse PCR quantitative en utilisant des séquences de marqueurs appropriées afin de calculer le nombre de copies de l'ADN génomique ;
b) la détermination de l'activité métabolique d'oeufs d'helminthes embryonnés à l'aide de méthodes de biochimie et/ou de biologie moléculaire, où afin de déterminer l'activité métabolique d'oeufs de *Trichuris* embryonnés, la teneur en ATP est mesurée et/ou les oeufs de *Trichuris* sont dans un premier temps traités à l'aide d'un agent de prétraitement choisi parmi l'acide hypochloreux, la chitinase et/ou la protéase puis sont colorés avec des sels de tétrazolium,
c) la détermination de la capacité d'induction de l'expression des gènes dans des oeufs d'heltminthes embryonnés,
d) la détermination par microscope de la motilité de larves d'helminthes se trouvant dans l'oeuf sur de longues périodes d'observation après incubation préalable à des températures élevées, et/ou

e) la détermination du taux d'éclosion de larves de *Trichuris* chez l'animal de laboratoire, où les oeufs embryonnés intacts récupérés dans le contenu de tractus digestif sont quantifiés en comparaison avec une norme interne.

2. Procédé selon la revendication 1a), où le nombre de copies de l'ADN génomique est déterminé au moyen d'une analyse PCR quantitative en utilisant des séquences appropriées, spécifiques au *Trichuris suis.*

3. Procédé selon la revendication 1b), **caractérisé en ce que** les oeufs de *Trichuris* sont incubés au préalable avant la mesure par luminescence dans les conditions qui suivent :

  aa) entre 36 °C et 42 °C ;
  bb) entre 2 et 30 heures ;
  cc) dans un milieu de suspension présentant une valeur pH comprise entre 0,1 et 3.

4. Procédé selon la revendication 1c), **caractérisé en ce que** la capacité d'induction d'une protéine de choc thermique est déterminée.

5. Procédé selon la revendication 1c) **caractérisé en ce que** l'expression est démontrée par hybridation avec une sonde nucléotidique à marquage fluorescent.

6. Procédé selon la revendication 1c), **caractérisé en ce que** l'hybridation est démontrée à l'aide de la cytométrie en flux.

7. Procédé selon la revendication 1d), **caractérisé en ce que** la motilité des larves d'helminthes se trouvant dans l'oeuf est déterminée par microscope sur des périodes allant de 2 minutes à 8 heures à l'aide d'images prises en accéléré.

8. Procédé selon la revendication 1e), **caractérisé en ce que** les oeufs d'helminthes à examiner sont marqués à l'aide des sonde fluorescentes et les normes internes sont marquées à l'aide de sondes fluorescentes présentant une autre couleur.

9. Procédé selon la revendication 1e), **caractérisé en ce que** le contenu du tractus digestif de lapins et/ou de porcs est utilisé en tant que système de contrôle.

10. Procédé selon la revendication 1e), caractérisé en ce des oeufs de *Trichuris* non embryonnés ou inactivés sont utilisés en tant que norme interne en vue du calcul du taux d'éclosion.

11. Procédé selon l'une quelconque des revendications précédentes, **caractérisé en ce qu'**au moins quatre déterminations choisies parmi les déterminations 1a), 1b), 1c), 1d) et/ou 1e) sont réalisées.

12. Procédé selon l'une quelconque des revendications précédentes, **caractérisé en ce que** les oeufs de *Trichuris* ont été incubés au préalable avant la réalisation de la détermination sur une période allant d'au moins 30 minutes à 24 heures selon des conditions normées avec précision, où l'incubation préalable inclut éventuellement également une modification d'un paramètre essentiel, notamment de la température.

```
                                               ┌► Forward Primer
TAGCAGCGACGGCAGGTGCCCGTCATCGCTGGCAGGCAGCCGGAG CTGCGGAGAGCGGCTAACT CAGC
  ------------------         ┌─────────────────────────────┐
GCAGTACGGAAGCTGCCCG AGTTGGCTACGTCGTCGCTACAT CGTCGTCAGCGTACAGCGCGACTGAG
  ------------------         └─────────────────────────────┘
     TaqMan-Probe                          Reverse Primer ◄──┘
```

Figur 1: Ausschnitt aus der ITS2-Sequenz von *T. suis* mit Primern und TaqMan-Probe für die quantitative PCR

$$\text{Luciferin + ATP + O}_2 \quad \xrightarrow[\text{Mg}^{2+}]{\text{Luciferase}} \quad \text{Oxyluciferin + AMP + PPi + h}\nu$$

Figur 2: Luciferase-Reaktion zum quantitativen Nachweis von Adenosintriphosphat

**Erläuterungen zu Fig. 3:**

- Aktiv ohne Preincubation:   Nicht inaktivierte Eier ohne Vorbehandlung bei 37°C über 19 Stunden
                              (Eier im Ruhezustand bei 2-8°C)
- Inaktiv ohne Preincubation:   Kryoinaktivierte Eier ohne Vorbehandlung bei 37°C über 19 Stunden
- Aktiv ohne Preincubation:   Nicht inaktivierte Eier mit Vorbehandlung bei 37°C über 19 Stunden
- Inaktiv ohne Preincubation:   Kryoinaktivierte Eier mit Vorbehandlung bei 37°C über 19 Stunden

Figur 3: Luciferase-Reaktion zum quantitativen Nachweis von Adenosintriphosphat

**Figur 4:** Standardkurve von Adenosintriphosphat

```
T.suis_aa            ------------IGRRYDDAAVQSDMKHWPPKVVSDGGKPKIQVEYKGETKMFTPEBVS 47
C.elegans_aa         RNPENTVPDAKRLIGRRFDEETVQSDIKHWPFTVKGKQGKPVVBVEVKGEKREPNAEBIS 120
                             ****:*: :****:*****.* .. *** ::** ***.: *..**:*

T.suis_aa            AMVLVKMKBTAEAYLG------------------------------------------- 63
C.elegans_aa         AMVLQKMKBTAEAVLGHSVRDAVITVPAYFNDSQRQATKDAATIAGLNAIRIINEPTAAA 180
                     **** ******** **
```

Figur 5: Vergleich der Proteinsequenz des putativen Hitzeschock-Proteins aus *T. suis* (Ausschnitt) mit hsp70 aus *C.elegans* (gleiche Basen:"*"; Basen mit ähnlichen physikochemischen Eigensschaften "." oder ":"):

```
CTCCGTCACTGACCACCTTGAAAGGCCAATGCTTCATGTCAGACTG
```

Figur 6: FISH-Sonde zum Nachweis der Expression von Hitzeschock-Protein mRNA in aktiven embryonierten Eiern von *T.suis* (T: Allyl-Amino-Thymin-Basen, an die Fluorophore gekoppelt sind)

Figur 7: Motilitätsindex von aktiven und thermisch inaktivierten *Trichuris suis* Eiern nach Inkubation bei 39°C

Figur 8: Motilitätsindex einer *Trichuris suis*-Präparation nach verschieden langen Beobachtungszeiträumen bei 39.5°C

Figur 9: Fluoreszenzmikroskopische Aufnahme von einer Mischung aus Rhodamin-X-markierten TSO und unmarkierten TSO als Kontrolle (links: Lichtmikroskopie; rechts: Fluoreszenzmikroskopie). Die Pfeile markieren fluoreszenzmarkierte TSO

Figur 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 199933479 A
- WO 2007076868 A
- WO 2007134761 A

**In der Beschreibung aufgeführte Nicht-Patentliteratur**

- **D M MCKAY.** *Parasitology,* 2006, vol. 132, 1-12
- **SUMMERS et al.** *Am J Gastroenterol,* vol. 98, 2034-2041
- **BEER.** *Br. Med. J.,* 1971, vol. 3, 41
- **SUMMERS et al.** *Am. J. Gastroenterol.,* 2003, vol. 98, 2034-2041
- **BEER.** *Parasitol.,* 1973, vol. 67, 253-262
- **KRINGEL et al.** *Veterinary Parasitology,* 2006, 132-139
- **JOHNSON et al.** *Int. J. for Parasitology,* 1998, 627-633
- **PECSON et al.** *Applied and Environmental Microbiology,* 2006, 7864-7872
- **KRINGEL et al.** *Vet. Parasitol.,* 2006, vol. 139, 132-139
- **JOHNSON et al.** *Intl. J. Parasitol.,* 1998, vol. 28, 627-633
- **KRINGEL et al.** *Vet. Parasitol.,* 2006, vol. 139 (1), 32-139
- **SUMMERS et al.** *Gastroenterology,* 2005, vol. 128, 825-832
- **CUTILLAS et al.** *Parasitol Res,* 2001, vol. 100, 383-389
- **PECSON et al.** *Appl.Envir.Microbiol.,* 2006, vol. 72, 7864-7872
- **BEER.** *Parasitol.,* 1973, vol. 67, 263-278
- *Parasitology,* 1973, vol. 67, 253-262

SEQUENCE LISTING

<110> Dr. Falk Pharma GmbH

<120> Verfahren zur Charakterisierung der biologischen Aktivitaet von Helminth-Eiern, nämlich von Trichuris Eiern

<130> T.suis

<160> 7

<170> PatentIn version 3.5

<210> 1
<211> 136
<212> DNA
<213> Trichuris suis

<400> 1

```
tagcagcgac ggcaggtgcc cgtcatcgct ggcaggcagc cggagctgcg gagagcggct      60

aactcagcgc agtacggaag ctgcccgagt tggctacgtc gtcgctacat cgtcgtcagc     120

gtacagcgcg actgag                                                     136
```

<210> 2
<211> 19
<212> DNA
<213> artificial sequence

<220>
<223> primer

<400> 2
```
ctgcggagag cggctaact          19
```

<210> 3
<211> 23
<212> DNA
<213> artificial sequence

<220>
<223> primer

<400> 3
```
agttggctac gtcgtcgcta cat          23
```

<210> 4
<211> 16
<212> DNA

<213> artificial sequences

<220>
<223> probe

<400> 4
cagtacggaa gctgcc          16

<210> 5
<211> 63
<212> PRT
<213> Trichuris suis

<400> 5

```
Ile Gly Arg Arg Tyr Asp Asp Ala Ala Val Gln Ser Asp Met Lys His
1               5                   10                  15
```

```
Trp Pro Phe Lys Val Val Ser Asp Gly Gly Lys Pro Lys Ile Gln Val
            20                  25                  30
```

```
Glu Tyr Lys Gly Glu Thr Lys Met Phe Thr Pro Glu Glu Val Ser Ala
            35                  40                  45
```

```
Met Val Leu Val Lys Met Lys Glu Thr Ala Glu Ala Tyr Leu Gly
        50                  55                  60
```

<210> 6
<211> 120
<212> PRT
<213> Caenorhabditis elegans

<400> 6

```
Arg Asn Pro Glu Asn Thr Val Phe Asp Ala Lys Arg Leu Ile Gly Arg
1               5               10              15

Arg Phe Asp Glu Glu Thr Val Gln Ser Asp Ile Lys His Trp Pro Phe
            20              25              30

Thr Val Lys Gly Lys Gln Gly Lys Pro Val Val Glu Val Glu Val Lys
            35              40              45

Gly Glu Lys Arg Glu Phe Asn Ala Glu Glu Ile Ser Ala Met Val Leu
    50              55              60

Gln Lys Met Lys Glu Thr Ala Glu Ala Val Leu Gly His Ser Val Arg
65              70              75              80

Asp Ala Val Ile Thr Val Pro Ala Tyr Phe Asn Asp Ser Gln Arg Gln
            85              90              95

Ala Thr Lys Asp Ala Ala Thr Ile Ala Gly Leu Asn Ala Ile Arg Ile
            100             105             110

Ile Asn Glu Pro Thr Ala Ala Ala
        115             120
```

<210> 7
<211> 46
<212> DNA
<213> artificial sequence

<220>
<223> probe

<400> 7
ctccgtcact gaccaccttg aaaggccaat gcttcatgtc agactg          46